# EUROPÄISCHE PATENTSCHRIFT

(11) **EP 0 561 738 B1**
(45) Veröffentlichungstag und Bekanntmachung des Hinweises auf die Patenterteilung: **04.06.1997**
(21) Anmeldenummer: 93810173.0
(22) Anmeldetag: 09.03.1993
(51) Int. Cl.: B41M 5/145, B41M 5/30, C07D 493/10

(54) **Fluoran Farbbildner**
Fluoran colour-formers
Composés chromogènes de fluorane

(30) Priorität: 17.03.1992 CH 862/92; 10.11.1992 CH 3475/92
(43) Veröffentlichungstag der Anmeldung: 22.09.1993
(73) Patentinhaber: Ciba Specialty Chemicals Holding Inc., 4057 Basel (CH)
(72) Erfinder: Zink, Rudolf, CH-4106 Therwil (CH)

(56) Entgegenhaltungen:
- EP-A- 0 451 766
- DE-A- 2 001 864
- DE-A- 2 634 400
- FR-A- 2 224 456
- US-A- 3 715 226
- US-A- 3 910 956

## Beschreibung

Es sind bereits zahlreiche Fluoran Farbbildner zur Verwendung in druck- oder wärmeempfindlichen Materialien bekannt geworden. An die Lagerstabilität dieser Materialien und an die Echtheit der mittels dieser Fluorane erzeugten Abbildungen werden hohe Anforderungen gestellt. Daher ist die Sublimationsechtheit und die Migrationsstabilität von Farbbildnern Gegenstand andauernder Forschungsbemühungen.

In der US-A-3,715,226 und der DE-OS-2,001,864 werden Fluoranfarbbildner für druckempfindliche Aufzeichnungsmaterialen offenbart, darunter auch die Verbindung 3-Diethylamino-7-carbomethoxyfluoran, 3-Diethylamino-7-Carboxyfluoran und 3-Diethylamino-5-methyl-7-benzoylfluoran.

Die vorliegende Erfindung betrifft Fluorane, Verfahren zu ihrer Herstellung und ihre Verwendung als Farbbildner in druckempfindlichen oder wärmeempfindlichen Aufzeichnungsmaterialien mit verbesserter Sublimationsechtheit und Migrationsstabilität.

Das druck- oder wärmeempfindliche Aufzeichungsmaterial enthält mindestens einen Farbbildner der Formel 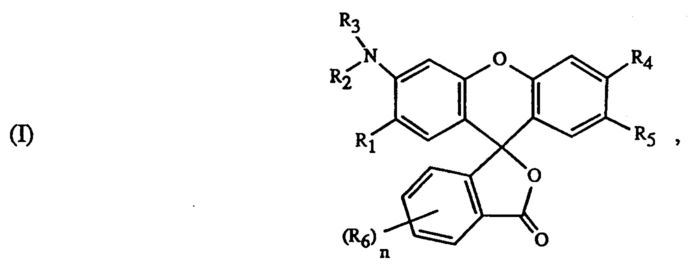 worin
- R₁: Wasserstoff oder C₁-C₄-Alkyl;
- R₂ und R₃: unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
- R₂ und R₃: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
- R₄: Wasserstoff, Hydroxy oder C₁-C₄-Alkyl;
- R₅: Nitro; SO₂R₇; SO₂OR₈; SO₂NR₉R₁₀; COR₁₁; CONR₉R₁₀; oder C₁-C₄-Halogenalkyl; nicht substituierter oder durch Halogen oder Hydroxy substituierter 2-Triazinyl-oder 1-Benztriazolylrest;
- R₆: Halogen; Nitro; C₁-C₄-Alkyl; C₁-C₄-Halogenalkyl; Amino; mono-C₁-C₄-Alkylamino; di-C₁-C₄-Alkylamino; oder COR₁₁;
- n: 0; 1; 2; 3; oder 4;
- R₇: C₁-C₈-Alkyl; oder C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-Alkyl;
- R₈: Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
- R₉ und R₁₀: unabhängig voneinander Wasserstoff; oder C₁-C₈-Alkyl; oder
- R₉ und R₁₀: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring; und
- R₁₁: Hydroxy; C₁-C₈-Alkyl; C₂-C₈-Alkoxy; C₁-C₈-Halogenalkyl; im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy
bedeutet,
mit der Massgabe, dass 3-Diethylamino-7-methylsulfonylfluoran, 3-Diethylamino-7-nitrofluoran, 3-Diethylamino-6-methyl-7-nitrofluoran, 3-Diethylamino-fluoran-7-sulfonsäure, 3-Diethylamino-7-(4-ethoxyphenyl)sulphonylfluoran und 3-Diethylamino-7-benzyloxyfluoran nicht mit umfaßt sind.

In der Literatur werden die einzelnen Substituentenposititionen im Fluoranring unterschiedlich numeriet. In der vorliegenden Anmeldung wird die folgende Numerierung verwendet:

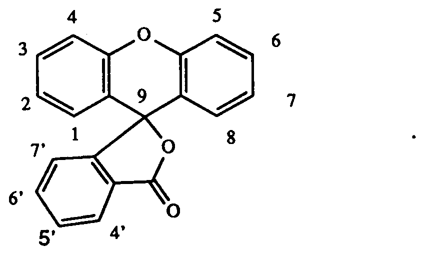

Im Rahmen der vorstehenden Definition haben die jeweiligen Substituenten oder Radikale insbesondere die folgenden Bedeutungen:

Halogen ist Fluor, Chlor oder Brom, insbesondere Fluor oder Chlor.

Alkyl bedeutet im Rahmen der jeweiligen Definition geradkettiges oder verzweigtes Alkyl. Beispiele solcher Alkylreste sind Methyl, Ethyl, n-Propyl, Isopropyl, n-Butyl, 1-Methylbutyl, sek.Butyl, tert.Butyl, n-Pentyl, Amyl, Isoamyl, n-Hexyl, 2-Ethyl-hexyl, n-Heptyl, n-Octyl, Isooctyl, 1,1,3,3-Tetramethylbutyl.

Beispiele für Halogenalkyl sind insbesondere die C₁-C₂-Halogenalkylreste, wie Trichlormethyl, Trifluormethyl, Dichlorfluormethyl, Difluorchlormethyl, Perchlorethyl, 1,1,2,2-Tetrachlorethyl, 1,1,2,2-Tetrafluorethyl, 2,2,2-Trichlorethyl. R₅ als C₁-C₈-Halogenalkyl sind insbesondere die zuvor genannten Halogenalkyle aber auch Alkylradikale, bei denen alle oder zumindest der überwiegende Teil der C-H Bindungen durch C-Cl oder C-F ersetzt ist.

Alkoxy ist insbesondere Methoxy, Ethoxy, n-Propoxy, Isopropoxy, n-Butoxy, Isobutoxy, sek.Butoxy und tert.Butoxy, C₁-C₄Alkoxy-C₁-C₄Alkyl insbesondere Methoxymethyl, Methoxyethyl, Ethoxymethyl oder Ethoxyethyl.

Mono-C₁-C₅-Alkylamino ist insbesondere Methylamino, Ethylamino, Propylamino, Butylamino und Pentylamino. Di-C₁-C₅-Alkylamino umfaßt sowohl die gemischten wie auch die gleichnamig substituierten Radikale, wie Methylethylamino, Dimehtylamino, Diethylamino, Methylpropylamino, Methylbutylamino, Di-n-propylamino, Di-isopropylamino, Di-n-butylamino und Di-n-pentylamino etc..

In Phenyl-C₁-C₄-Alkyl und Phenyl-C₁-C₄-Alkoxy kann der Phenylrest über eine geradkettige oder verzweigte Alkyl- bzw. Alkoxykette gebunden sein. Bevorzugt ist Phenethyl, Benzyl und Phenylmethoxy.

Der Phenylrest in Phenyl-C₁-C₄-Alkyl, Phenyl-C₁-C₄-Alkoxy bzw. Phenyl ist vorzugweise unsubstituiert oder bis zu dreifach gleich oder verschieden durch die genannten Substituenten substituiert.

Beispiele für C₃-C₅-Alkenyl sind Allyl, 1-Propenyl oder 2-Pentenyl, Isopropenyl oder 2-Butenyl. Allyl ist bevorzugt. C₄-C₇-Cyclohexyl bedeutet Cyclobutyl, Cycloheptyl, Cyclohexyl oder Cycloheptyl, wobei Cyclohexyl im Vordergrund des Interesses steht.

Die Verbindungen der Formel (I) sind normalerweise farblos oder höchstens schwach gefärbt. Wenn diese sublimationsechten Farbbildner mit einem vorzugsweise sauren Entwickler, d.h. einem Elektronenakzeptor, in Kontakt gebracht werden, so ergeben sie je nach der Bedeutung der Substituenten R₁ bis R₆ und dem verwendeten Entwickler intensiv orange oder rote Farbtöne. Die Verbindungen der Formel (I) sind auch sehr wertvoll im Gemisch mit einem oder mehreren anderen bekannten Farbbildnern, wie z.B. 3,3-(Bisaminophenyl-)-phthaliden, wie CVL, 3-Indolyl-3-aminophenyl- aza- oder -diazaphthaliden, (3,3-Bis-indolyl-)-phthaliden, 3-Aminofluoranen, 3,7-Diaminofluoranen, 3,7-Diamino-6-methyl-fluoranen, 3,6-Bisalkoxyfluoranen, 3,6-Bisdiarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um blaue, marineblaue, graue oder schwarze Färbungen zu ergeben.

Bevorzugt als Mischpartner sind Farbbildner, die eine schwarze Nuance ergeben, wie insbesondere 3,7-Diaminofluorane.

Die Verbindungen der Formel I werden zum Erzielen von blauen, marineblauen, grauen oder schwarzen Färbungen in einem für die gewünschte Farbtiefe geeigneten Mischungsverhältnis mit den anderen Farbbildnern eingesetzt. Die Mischungsverhältnisse werden auch durch den bei der Farbreaktion verwendeten Entwickler beeinflußt. Sie können in einfachen Serienuntersuchungen festgestellt werden. Vorzugsweise werden in den Farbmischungen von 10 bis 70 % des Fluorans der Formel I (Angaben in % bezogen auf die Mengen der eingesetzten Farbbildner) eingesetzt. Besonders bevorzugt sind etwa 30 bis 60 %. Derartige Mischungen kommen sowohl für Reaktionsdurchschreibepapiere als auch für Thermodruckpapiere in Frage. Im folgenden umfaßt der Begriff "Farbbildner" die Farbbildner der Formel I, wie auch deren Mischungen.

Die Verbindungen der Formel (I) zeigen sowohl auf aktivierten Tonen, wie auch auf phenolischen Unterlagen eine ausgezeichnete Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH-stabil und in den Kapselölen hervorragend löslich sind. Nach Belichtung in CB-Blatt weisen sie eine geige Abnahme der Farbstärke (CB-Desaktivierung) auf.

Ein druckempfindliches Material besteht beispielsweise aus mindestens einem Paar von Blättern, die mindestens einen Farbbildner der Formel (I) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxid, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-(α,α-dimethylbenzyl)-salicylsäure oder 3,5-Bis-(α-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein. Die Zinksalicylate sind z.B. in EP-A-0,181,283 oder DE-A-2,242,250 beschrieben.

Die Entwickler können zusätzlich auch im Gemisch mit an sich unreaktiven oder wenig reaktiven Pigmenten oder weiteren Hilfsstoffen wie Kieselgel oder UV-Absorbern, wie z.B. 2-(2'-Hydroxyphenyl-)benztriazolen eingesetzt werden. Beispiele für solche Pigmente sind: Talk, Titandioxid, Aluminiumoxid, Aluminiumhydroxid, Zinkoxid, Kreide, Tone wie Kaolin, sowie organische Pigmente, z.B. Harnstoff-Formaldehydkondensate (BET-Oberfläche 2-75 m²/g) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmäßig erzielt werden, indem man die Farbbildner in schaum-, schwamm- oder bienenwabenartigen Strukturen einarbeitet. Vorzugsweise sind die Farbbildner in Mokierkapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes, mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester wie z.B. Tricresylphosphat, Di-n-butylphthalat, Dioctylphthalat, Trichlorethylphosphat, aromatische Ether wie Benzylphenylether, Kohlenwasserstofföle, wie Paraffin oder Kerosin, aromatische Kohlenwasserstoffe, z.B. mit Isopropyl, Isobutyl, sek.Butyl oder tert.Butyl alkylierte Derivate von Diphenyl, Naphthalin oder Terphenyl, Dibenzyltoluol; partiell hydriertes Terphenyl, mono-, bis- oder tetra-C₁-C₃-alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Phthalide dadurch aus, dass sie gut löslich sind und pH-Beständigkeit z.B. in einem pH-Bereich von 4 bis 10 zeigen.

Die Kapselwände können durch Koazervationskräfte gleichmäßig um die Tröpfchen der Farbbildnerlösung herum gebildet werden, wobei das Einkapselungsmaterial z.B. in der US-Patentschrift 2,800,457 beschrieben ist. Die Kapseln können vorzugsweise auch aus einem Aminoplast oder modifizierten Aminoplasten durch Polykondensation gebildet werden, wie es in den britischen Patentschriften 989,264, 1,156,725, 1,301,052 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid oder Polyurethan.

Die Farbbildner der Formel (I) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Mit Vorteil können die erfindungsgemässen Farbbildner der Formel I als Mischungskomponenten in mikroverkapselte Farbbildnersystemen eingesetzt werden. Dabei ist die Sublimationsechtheit der erfindungsgemässen Fluorane der Formel I von besonderem Vorteil. Die aus dem Stand der Technik bekannten gelb- bzw. orange-schreibenden Fluorane neigen, wie eingangs erwähnt, eher zur Sublimation bzw. Migration als die anderen in derartigen Mischungen eingesetzten Farbbildner. Das Mischungsverhältnis muß deshalb den Eigenschaften der am ehesten sublimierenden Komponente solcher Mischungen Rechnung tragen. Demgegenüber entspricht die Sublimationsechtheit der erfindungsgemässen Verbindungen denen der übrigen Mischungskomponenten. Die daraus resultierenden Vorteile liegen auf der Hand: bei der Auswahl der Mischungskomponenten muss keine besondere Rücksicht auf leichter sublimierbare Bestandteile des Farbbildnersystems genommen werden. Die Verbesserung der Migrationsstabilität in den erfindungsgemässen Farbmischungen wirkt sich insbesondere vorteilhaft auf das entwickelte Bild aus. Auch nach längerem Lagern des Kopiermaterials bleibt das Bild an seinen Rändern scharf und verschwimmt nicht.

Bevorzugt wird eine Anordnung, bei der der eingekapselte Farbbildner in Form einer Schicht auf der Rückseite eines Übertragungsblattes und der Elektronenakzeptor (Farbentwickler) in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo-oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Vorzugsweise weist das Durchschreibematerial auch eine kapselfreie, den Farbbildner enthaltende Schicht und eine farbentwickelnde Schicht auf, die als Farbentwickler mindestens ein anorganisches Metallsalz eines mehrwertigen Metalls, vor allem Halogenide oder Nitrate, wie z.B. Zinkchlorid, Zinnchlorid, Zinknitrat oder deren Gemische enthält.

Die Verbindungen der Formel (I) können auch als Farbbildner in einem thermoreaktiven Aufzeichnungsmaterial verwendet werden. Dieses enthält in der Regel mindestens einen Schichtträger, einen oder mehrere Farbbildner, einen Elektronenakzeptor und gegebenenfalls auch ein Bindemittel und/oder Wachs. Gewünschtenfalls können auch Aktivatoren oder Sensibilisatoren im Aufzeichnungsmaterial vorhanden sein.

Mit Vorteil können die erfindungsgemässen Farbbildner der Formel I als Mischungskomponenten in thermoreaktiven Farbbildnersystemen eingesetzt werden. Dabei ist die Lagerstabilität (heiss und/oder feucht) und die geringe Hintergrundverfärbung der erfindungsgemässen Fluorane von besonderem Vorteil.

Thermoreaktive Aufzeichnungssysteme umfassen, z.B. wärmeempfindliche Aufzeichnungs-und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren wie sie in druckempfindlichen Papieren verwendet werden. Beispiele für Entwickler sind die bereits erwähnten Tonminerale und Phenolharze oder auch phenolische Verbindungen, wie sie beispielsweise in der DE-PS-1,251,348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäuremethyl- oder -benzylester, 4-Hydroxydiphenylsulfon, 4'-Hydroxy-4-methyldiphenylsulfon, 4'-Hydroxy-4-isopropoxydiphenylsulfon, 4,4'-Cyclo-hexylidendiphenol, 4,4'-Isopropylidendiphenol, 4,4'-Isopropyliden-bis-(2-methylphenol), ein Antipyrinkomplex von Zinkthiocyanat, ein Pyridinkomplex von Zinkthiocyanat, 4,4-Bis(4-hydroxyphenyl)valeriansäure, Hydrochinon, Pyrogallol, Phloroglucin, p-, m-, o-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Farbbildner und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol verwendet werden. Die bevorzugte Anordnung ist jedoch diejenige, bei der der Farbbildner und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten z.B. Talk, Titandioxid, Zinkoxid, Aluminiumhydroxid, Calciumcarbonat (z.B. Kreide), Tone oder auch organische Pigmente, wie z.B. Harnstoff-Formaldehydpolymerisate enthalten. Um die Farbbildung nur innerhalb eines begrenzten Temperaturbereiches zu bewirken, können Substanzen wie Harnstoff, Thioharnstoff, Diphenylthioharnstoff, Acetamid, Acetanilid, Benzolsulfanilid, Stearinsäureamid, Bisstearoylethylendiamid, Phthalsäureanhydrid, Metallstearate, wie z.B. Zinkstearat, Phthalsäurenitril, Dimethylterephthalat, Dibenzyltherephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formel (I) ist die Herstellung eines Farbbildes mittels photohärtbarer Mikrokapseln, wie sie z.B. in der DE-OS-3,247,488 beschrieben sind.

Bevorzugt sind Farbbildnersysteme, die als Mischungskomponenten mindestens ein Fluoran der Formel I enthalten, worin
- R₁: Wasserstoff oder C₁-C₄-Alkyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten oder durch C₁-C₄-Alkyl substituierten Pyrrolidin- oder Piperidin-Ring;
- R₄: Wasserstoff oder C₁-C₄-Alkyl;
- R₅: Nitro; SO₂R₇; SO₂OR₈; SO₂NR₉R₁₀; COR₁₁; CONR₉R₁₀; oder C₁-C₄-Halogenalkyl;
- n: 0, 1, 2, 3 oder 4;
- R₆: wenn n 1, 2, 3 oder 4 ist, Halogen; wenn n 1 oder 2 ist, C₁-C₄-Alkyl, C₁-C₄-Halogenalkyl; oder wenn n 1 ist, Nitro, COR₁₁, Amino, mono-C₁-C₄-Alkylamino oder di-C₁-C₄Alkylamino;
- R₇: C₁-C₄-Alkyl; oder C₁-C₄-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₂-alkyl;
- R₈: Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
- R₉ und R₁₀: unabhängig voneinander Wasserstoff; oder C₁-C₄-Alkyl; oder
- R₉ und R₁₀: gemeinsam mit dem Stickstoffatom, an weiches sie gebunden sind, einen unsubstituierten oder durch C₁-C₄-Alkyl substituierten Pyrrolidin- oder Piperidin-Ring; und
- R₁₁: C₁-C₄-Alkyl; C₁-C₄-Halogenalkyl; C₂-C₄-Alkoxy; im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy
bedeutet.

Hervorzuheben sind Farbbildnersysteme, die als Mischungskomponenten mindestens ein Fluoran der Formel I enthalten,
worin
- R₁: Wasserstoff oder Methyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten Pyrrolidin- oder Piperidin-Ring;
- R₄: Wasserstoff oder Methyl;
- R₅: Nitro; SO₂R₇; SO₂NR₉R₁₀; COR₁₁; CONR₉R₁₀; oder C₁-C₄-Halogenalkyl;
- n: 0, 1, 2, 3 oder 4;
- R₆: wenn n 1, 2, 3 oder 4 ist, Halogen; wenn n 1 oder 2 ist, Methyl; oder wenn n 1 ist, Nitro, Amino, mono-C₁-C₄-Alkylamino oder di-C₁-C₄-Alkylamino;
- R₇: C₁-C₄-Alkyl; oder C₁-C₄-Halogenalkyl; unsubstituiertes oder im Phenylrest durch Halogen , C₁-C₄-Alkyl, oder C₁-C₄-Alkoxy, substituiertes Phenyl;
- R₉ und R₁₀: unabhängig voneinander Wasserstoff; oder C₁-C₄-Alkyl;
- R₁₁: C₁-C₄-Alkyl; C₂-C₄-Alkoxy; im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
bedeutet.

Insbesondere betrifft die Erfindung Farbbildnersysteme der Formel I, der bevorzugten Formel I, bzw. der hervorgehobenen Formel I, worin R₅ SO₂R₇, SO₂OR₈, SO₂NR₉R₁₀, COR₁₁, CONR₉R₁₀, C₁-C₄-Halogenalkyl oder Nitro bedeutet,

Unter den genannten Verbindungen der Formel I sind diejenigen bevorzugt, bei denen R₅ COR₁₁ bedeutet und ganz besonders diejenigen, bei denen R₁₁ C₁-C₄-Alkyl, C₁-C₄-Alkoxy oder Phenyl-C₂-C₄-Alkoxy bedeutet Im Vordergrund des Interesses stehen dabei Verbindungen, bei denen R₁₁ C₂-C₄-Alkoxy oder Phenyl-C₁-C₄-Alkoxy bedeutet.

Die Verbindungen der Formel I sind teilweise neu. Aus der Literatur sind wenige Verbindungen der Formel I beschrieben: 3-Diethylamino-7-methylsulfonylfluoran in der DE-A-2,155,986, 3-Diethylamino-7-nitrofluoran, 3-Diethylamino-6-methyl-7-nitrofluoran, 3-Diethylamino-7-methoxycarbonylfluoran in der US-A-3,637,757, 3-Diethylamino-7-formylfluoran in der DE-A-2,001,864 und 3-Diethylamino-fluoran-7-sulfonsäure in GB-B-1,418,871. In keiner dieser Literaturstellen werden Farbbildnermischungen erwähnt.

Die erfindungsgemässen neuen Fluorane entsprechen der allgemeinen Formel 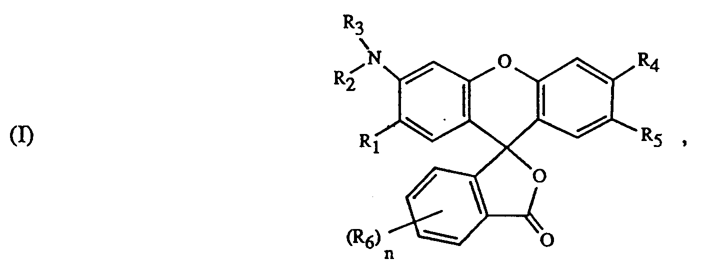 worin
- R₁: Wasserstoff oder C₁-C₄-Alkyl;
- R₂ und R₃: unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
- R₂ und R₃: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
- R₄: Wasserstoff, Hydroxy oder C₁-C₄-Alkyl;
- R₅: Nitro; SO₂R₇; SO₂OR₈; SO₂NR₉R₁₀; COR₁₁; CONR₉R₁₀; oder C₁-C₄-Halogenalkyl; nicht substituierter oder durch Halogen oder Hydroxy substituierter 2-Triazinyl-oder 1-Benztriazolylrest;
- R₆: Halogen; Nitro; C₁-C₄-Alkyl; C₁-C₄-Halogenalkyl; Amino; mono-C₁-C₄-Alkylamino; di-C₁-C₄-Alkylamino; oder COR₁₁;
- n: 0; 1; 2; 3; oder 4;
- R₇: C₁-C₈-Alkyl; oder C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-Alkyl;
- R₈: Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
- R₉ und R₁₀: unabhängig voneinander Wasserstoff; oder C₁-C₈-Alkyl; oder
- R₉ und R₁₀: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring; und
- R₁₁: Hydroxy; C₁-C₈-Alkyl; C₂-C₈-Alkoxy; C₁-C₈-Halogenalkyl; im Phenylrest durch Halogen, C₁-C₄-Alkyl, C₁-C₄-Halogenalkyl, C₁-C₄-Alkoxy substituiertes Phenyl; Phenyl-C₁-C₄-Alkyl; oder Phenyl-C₁-C₄-Alkoxy;
bedeutet, mit der Massgabe, dass 3-Diethylamino-7-methylsulfonylfluoran, 3-Diethylamino-7-nitrofluoran, 3-Diethylamino-6-methyl-7-nitrofluoran, und 3-Diethylamino-fluoran-7-sulfonsäure nicht mit umfaßt sind.

Bevorzugt sind Verbindungen der Formel I
worin
- R₁: Wasserstoff oder C₁-C₄-Alkyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten oder durch C₁-C₄-Alkyl substituierten Pyrrolidin- oder Piperidin-Ring;
- R₄: Wasserstoff oder C₁-C₄-Alkyl;
- R₅: Nitro; SO₂R₇; SO₂OR₈; SO₂NR₉R₁₀; COR₁₁; CONR₉R₁₀; oder C₁-C₄-Halogenalkyl;
- n: 0, 1, 2, 3 oder 4;
- R₆: wenn n 1,2,3 oder 4 ist, Halogen; wenn n 1 oder 2 ist, C₁-C₄-Alkyl, C₁-C₄-Halogenalkyl; oder wenn n 1 ist, Nitro, COR₁₁, Amino, mono-C₁-C₄-Alkylamino oder di-C₁-C₄Alkylamino;
- R₇: C₁-C₄-Alkyl; oder C₁-C₄-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₂-alkyl;
- R₈: Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
- R₉ und R₁₀: unabhängig voneinander Wasserstoff; oder C₁-C₄-Alkyl; oder
- R₉ und R₁₀: gemeinsam mit dem Stickstoffatom, an welches sie gebunden sind, einen unsubstituierten oder durch C₁-C₄-Alkyl substituierten Pyrrolidin- oder Piperidin-Ring; und
- R₁₁: C₁-C₄-Alkyl; C₁-C₄-Halogenalkyl; C₂-C₄-Alkoxy; im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy
bedeutet.

Besonders bevorzugt sind Fluorane der Formel I,
worin
- R₁: Wasserstoff oder C₁-C₄-Alkyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten oder durch C₁-C₄-Alkyl substituierten Pyrrolidin- oder Piperidin-Ring;
- R₄: Wasserstoff oder C₁-C₄-Alkyl;
- R₅: Nitro; SO₂R₇; SO₂OR₈; SO₂NR₉R₁₀; COR₁₁; CONR₉R₁₀; oder C₁-C₄-Halogenalkyl;
- n: 0, 1, 2, 3 oder 4;
- R₆: wenn n 1, 2, 3 oder 4 ist, Halogen; wenn n 1 oder 2 ist, Nitro, C₁-C₄-Alkyl, C₁-C₄-Halogenalkyl; oder wenn n 1 ist, COR₁₁, Amino, mono-C₁-C₄-Alkylamino oder di-C₁-C₄Alkylamino;
- R₇: C₁-C₄-Alkyl; oder C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
- R₈: Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
- R₉ und R₁₀: unabhängig voneinander Wasserstoff; oder C₁-C₄-Alkyl; oder
- R₉ und R₁₀: gemeinsam mit dem Stickstoffatom, an welches sie gebunden sind, einen unsubstituierten oder durch C₁-C₄-Alkyl substituierten Pyrrolidin- oder Piperidin-Ring; und
- R₁₁: C₁-C₄-Alkyl; C₁-C₄-Halogenalkyl; C₂-C₄-Alkoxy; im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy;
bedeutet.

Hervorzuheben sind Fluorane der Formel I,
worin
- R₁: Wasserstoff oder Methyl;
- R₂ und R₃: unabhängig voneinander Wasserstoff; C₁-C₅-Alkyl; oder
- R₂ und R₃: gemeinsam mit dem Stickstoffatom, an welches sie gebunden sind, einen unsubstituierten Pyrrolidin- oder Piperidin-Ring;
- R₄: Wasserstoff oder Methyl;
- R₅: Nitro; SO₂R₇; SO₂NR₉R₁₀; COR₁₁; CONR₉R₁₀; oder C₁-C₄-Halogenalkyl;
- n: 0, 1, 2, 3 oder 4;
- R₆: wenn n 1, 2, 3 oder 4 ist, Halogen; wenn n 1 oder 2 ist, Nitro, Methyl; oder wenn n 1 ist, Nitro, Amino, mono-C₁-C₄-Alkylamino oder di-C₁-C₄-Alkylamino;
- R₇: C₁-C₄-Alkyl; oder C₁-C₄-Halogenalkyl; unsubstituiertes oder im Phenylrest durch Halogen , C₁-C₄-Alkyl, oder C₁-C₄-Alkoxy, substituiertes Phenyl;
- R₉ und R₁₀: unabhängig voneinander Wasserstoff; oder C₁-C₄-Alkyl;
- R₁₁: C₁-C₄-Alkyl; C₂-C₄-Alkoxy; im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder C₁-C₂-Alkoxy
bedeutet.

Insbesondere betrifft die Erfindung Farbbildnersysteme der Formel I, der bevorzugten Formel I, bzw. der hervorgehobenen Formel I, worin R₅ SO₂R₇, SO₂OR₈, SO₂NR₉R₁₀, COR₁₁, CONR₉R₁₀, C₁-C₄-Halogenalkyl oder Nitro bedeutet, und
- R₂ und R₃: unabhängig voneinander Wasserstoff oder C₄-C₈-Alkyl;
- R₂ und R₃: gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring bedeutet.

Unter den genannten Verbindungen der Formel I sind diejenigen bevorzugt, bei denen R₅ COR₁₁ bedeutet und ganz besonders diejenigen, bei denen R₁₁ C₁-C₄-Alkyl, C₂-C₄-Alkoxy oder Phenyl-C₁-C₄-Alkoxy bedeutet. Im Vordergrund des Interesses stehen dabei Verbindungen, bei denen R₁₁ C₂-C₄-Alkoxy oder Phenyl-C₁-C₄-Alkoxy bedeutet.

Die Verbindungen der Formel I können nach an sich bekannten Verfahren hergestellt werden. Die Erfindung betrifft auch Verfahren zur Herstellung von Verbindungen der Formel I,
a) gekennzeichnet durch die Umsetzung eines Benzophenons der Formel II mit einem Phenol oder Phenolether der Formel III 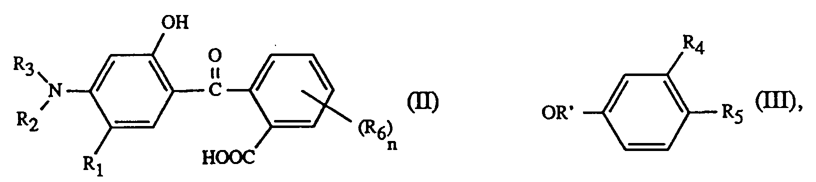 worin die Reste R₁ bis R₆ und n wie zuvor definiert sind und R' Wasserstoff oder C₁-C₄-Alkyl bedeutet, bei Temperaturen von vorzugsweise 0 bis 70° C, in 50 bis 100 %iger Schwefelsäure zu einem Phthalid der Formel 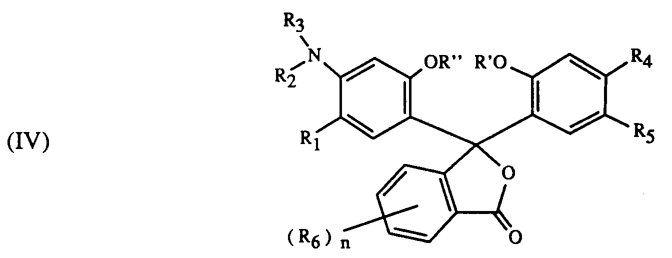 und anschliessender Cyclisierung bei Temperaturen von 20 bis 100° C zu einer Verbindung der Formel I.

Die Ausgangsverbindungen der Formeln (II) und (III) sind teilweise literaturbekannt oder können analog zu an sich bekannten Verfahren hergestellt werden. So sind etwa geeignete Verbindungen der Formel (III) aus der DE-A-4,029,131 oder der US-A-1,939,416 bekannt. Verfahren zu deren Herstellung werden auch in Houben Weyl, Methoden der organischen Chemie Band IX, S. 231 ff beschrieben.

Weiterhin können Verbindungen der Formel I durch Derivatisierungsreaktionen aus anderen Verbindungen der Formel I hergestellt werden.

So können z.B. die Sulfone oder die Sulfonamide der Formel I (R₅ = SO₂R₇ oder SO₂NR₉R₁₀) durch Umsetzung der Sulfonester der Formel I (R₅ = SO₂OR₈) mit Carbanionen oder Grignard Reagenzien (R₇⁻ oder R₇Mg-Halogen) oder Aminen (HNR₉R₁₀) hergestellt werden.

Die Erfindung betrifft somit auch Verfahren zur Herstellung von Sulfonen der Formel I,
worin
- R₅: SO₂R₇ bedeutet,
dadurch gekennzeichnet, dass man einen Sulfonester der Formel I,
worin
- R₅: SO₂OR₈ bedeutet,
mit einem Carbanion oder Grignard Reagenz der allgemeinen Formel V

R₇⁻ M⁺ oder R₇Mg-Hal (V),

worin
- M⁺: ein Kationenäquivalent eines Alkali- oder Erdalkalimetallions; und
- Hal: Halogen
bedeutet und
- R₇: wie zuvor definiert ist, umsetzt,
und
ein Verfahren zur Herstellung von Sulfonamiden der Formel I,
worin
- R₅: SO₂NR₉R₁₀bedeutet,
dadurch gekennzeichnet, dass man einen Sulfonester der Formel I,
worin
- R₅: SO₂OR₈ bedeutet,
mit einem Amin der Formel VI

HNR₉R₁₀ (VI),

worin
- R₉ und R₁₀: wie zuvor definiert sind, umsetzt.

Die folgenden Beispiele erläutern die Erfindung. Die angegebenen Prozentsätze beziehen sich, wenn nichts anderes angegeben ist, auf das Gewicht.
Herstellungsbeispiel 1:3-Diethylamino-7-butylsulfonyl-fluoran
   Zu 18,8 g 4'-Diethylamino-2'-hydroxybenzophenon-2-carbonsäure in 120 ml 98 %-iger Schwefelsäure werden bei 15 - 30° C innerhalb 30 min 14,5 g 1-Ethoxy-4(butylsulfonyl)benzol eingetragen und 24 Stunden bei 20 - 25° C gerührt. Anschliessend wird das Reaktionsgemisch auf eine Mischung aus 510 ml 40 %-iger Natronlauge, 550 ml Wasser und 250 ml Toluol ausgetragen, wobei eine Endtemperatur von 83° C erreicht wird. Es wird eine Stunde bei 80 - 83° C weitergerührt, die Toluolphase abgetrennt und zweimal mit 200 ml Wasser gewaschen. Nach Abdestillieren des Toluols isoliert man 29,1 g der Titelverbindung, die durch Umkristallisieren aus Isopropanol gereinigt wird. Man erhält so die gereinigte Verbindung der Formel 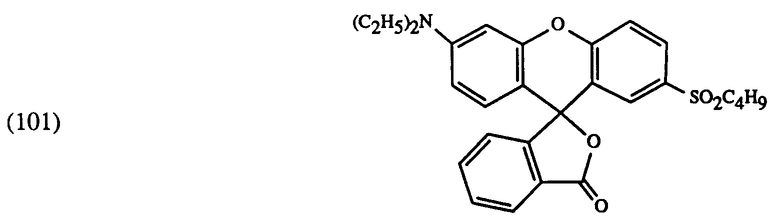 als farblose Kristalle vom Smp. 134 - 136° C.
   Die Verbindung ergibt mit handelsüblichen CF-Papieren einen orangen Farbton. Eine Migration aus CB-Papieren (Kapseln) kann nicht festgestellt werden.
Herstellungsbeispiel 2: 3-Diethylamino-7-p-tolylsulfonyl-fluoran
   31,3 g 4'-Diethylamino-2'-hydroxybenzophenon-2-carbonsäure werden in 200 ml 98 %-iger Schwefelsäure bei 34 - 40° C gelöst. Sodann werden bei 30° C portionsweise 24,8 g 4-Hydroxy-4'-methyldiphenylsulfon eingetragen und 20 Stunden bei 20 - 25° C nachgeführt. Die so erhältliche rote Lösung wird auf 1000 g Eis/Wasser ausgetragen und vom gut filtrierbaren Rückstand abgetrennt. Das Nutschgut wird mit Wasser gewaschen, in 200 ml 4 %-ige Natronlauge und 600 ml Toluol aufgenommen und eine Stunde bei 85° C gerührt. Danach wird die Toluolphase abgetrennt, zweimal mit je 100 ml warmen Wasser gewaschen und durch Destillation aufkonzentriert, bis das Produkt auskristallisiert. Nach dem Erkalten wird abfiltriert, mit etwas Isopropanol gewaschen und getrocknet. Man isoliert 27,2 g der Verbindung der Formel 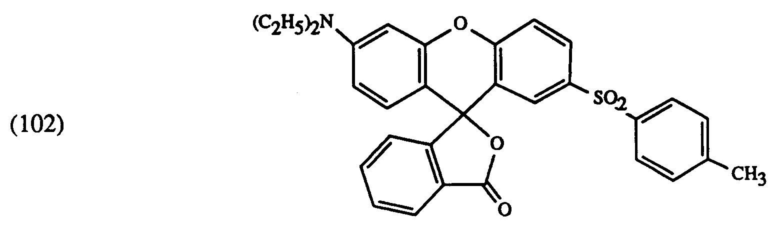 als farblose Kristalle vom Smp. 221 - 222° C.
   Auf Aktivton ergibt die Verbindung eine orange Farbe.
Herstellungsbeispiel 3: 3-Diethylamino-7-ethoxycarbonylfluoran
   a. via Säure
      313 g 4'-Diethylamino-2'-hydroxybenzophenon-2-carbonsäure werden in 830 g 98%iger Schwefelsäure bei ca. 40°C gelöst. Dann werden in ca. 30 Minuten 138 g 4-Hydroxybenzoesäure eingetragen und die Temperatur auf 80°C erhöht und während 4 Stunden bei dieser Temperatur gehalten. Die Kondensationslösung trägt man innerhalb ca. 2 Stunden auf 2000 ml Wasser/Eis aus, wobei der pH-Wert durch gleichzeitiges Zudosieren von ca. 1500 ml 10 N Natronlauge zwischen 1 und 4 gehalten wird. Durch Eiszugabe wird die Temperatur bei maximal 35°C gehalten. Das Volumen beträgt 4500 ml, die Suspension wird nach 1 Stunde bei 30°C filiert und mit ca. 3 l Wasser von 30°C gewaschen. Man erhält 1600 g eines wasserfeuchten Zwischenproduktes der Formel 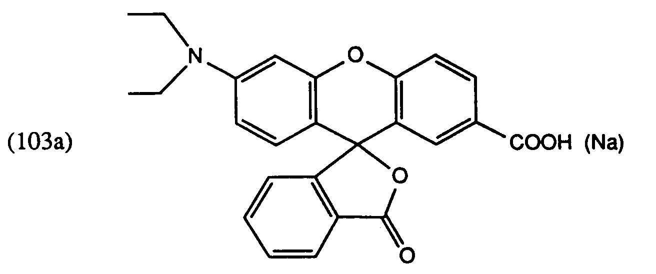 welches nach Trocknung 440 g wiegt.
      1500 g des wasserfeuchten Zwischenproduktes (103a) werden in 200 ml Toluol und 5g Tetrabutylammoniumbromid bei ca. 70°C suspendiert. Man kühlt auf 40°C und gibt 308 g Diethylsulfat hinzu. Unter kräftigem Rühren hält man die Temperatur während 3 Stunden bei 40 bis 45°C, wobei der pH-Wert mit Natronlauge bei ca. 11 gehalten wird. Anschliessend wird auf 70°C erwärmt, 1 Stunde bei 70 bis 75°C gehalten. Sodann werden die Phasen abgetrennt. Die Toluolphase wird mit Wasser gewaschen. Man destilliert 700 ml Toluol unter reduziertem Druck ab, filtert über Aktivkohle und versetzt bei 60°C mit 500 ml Methanol. Das Produkt kristallisiert nach Rühren in der Kälte vollständig aus, wird filtriert, mit 400 ml Methanol gewaschen und getrocknet. Man erhält 236,5 g des farblosen Produktes der Formel 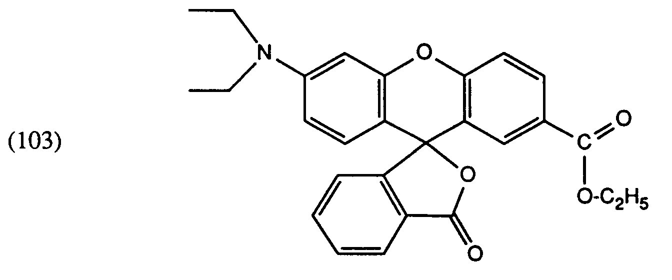
      Schmelzpunkt: 151-153°C
      Das Produkt löst sich hervorragend in den handelsüblichen Kapselölen, zeigt keine Migration aus den Kapseln und erzeugt auf den handelsüblichen CF-Papieren sowie in der Thermoapplikation ein intensives Orange.
   b. direkte Synthese
      31,3 g 4'-Diethylamino-2'hydroxybenzophenon-2-carbonsäure werden bei 35-40°C in 45 ml 100%iger Schwefelsäure (Monohydrat) gelöst. Man kühlt auf 10°C und trägt innerhalb von 30 Minuten 16,6 g 4-Hydroxybenzoesäureethylester ein, lässt auf 23°C erwärmen und rührt während 20 Stunden bei 23-28°C. Die Reaktionsmasse wird äuf 800 ml Eis/Wasser ausgetragen und nach 1 Stunde bei 22°C filtriert und mit 500 ml Wasser gewaschen. Man erhält 93 g eines feuchten Produktes der Formel (103), welches noch teilweise als Phthalid vorliegt.
      Das Nutschgut wird sodann mit 800 ml Toluol angeschlämmt, mit 21,4 g Natriumcarbonat versetzt und bei 75°C während 15 Minuten verrührt. Unter reduziertem Druck wird das Wasser azeotrop abdestilliert, die Toluollösung filtriert und zur Trockne eingeengt. Man erhält 28,6 g Rohprodukt der Formel (103), welches nach Umkristallisation einen Schmelzpunkt von 151-153°C aufweist und die gleichen applikatorischen Eigenschaften zeigt wie das unter 3a. hergestellte Produkt.
Herstellungsbeispiel 4: 7,8 g 4'-Diethylamino-2'-hydroxybenzophenon-2-carbonsäure werden in 50 ml 98%ger Schwefelsäure bei 35°C gelöst. Bei 20-25°C werden dann 8,9 g 2-(4,6-Diphenyl-1,3,5-triazin-2-yl)-5-methoxyphenol zugegeben und 24 Stunden bei 20-25°C gerührt. Die entstandene Lösung wird bei 80°C auf eine Mischung von 230 ml Wasser, 210 ml 10 N Natronlauge und 550 ml Toluol ausgetragen und während 30 Minuten bei 80-85°C gerührt. Man trennt die Wasserphase ab, wäscht mit warmem Wasser und lässt die klärfiltrierte Toluollösung unter Rühren abkühlen. Nach 2 Stunden wird bei 20°C filtriert und getrocknet. Man erhält so 12 g farblose Kristalle der Formel 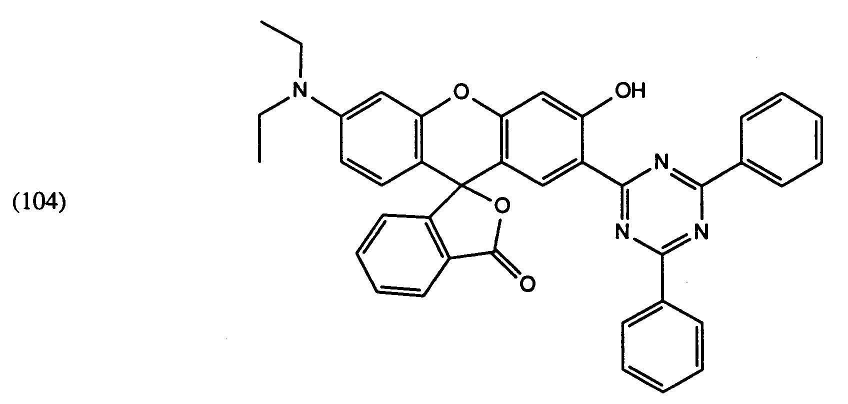
   Smp.: > 270°C
   Auf handelsüblichem CF-Papier wird damit eine rote Farbe erzeugt, welche eine gute Lichtechtheit aufweist.
Herstellungsbeispiel 5: 800 g feuchtes Nutschgut der Formel (103a) werden mit 10 N Natronlauge und 1 g Aliquat 336 angeschlämmt. Man erwärmt auf 85°C und tropft in die erhaltene lösung (pH 10,5) in einer Stunde bei 85-89°C 80 ml Benzylbromid zu. Man hält weitere vier Stunden bei dieser Temperatur, lässt dann auf 80°C sinken, gibt 500 ml Toluol zu und trennt die Wasserphase ab. Die Toluolphase wird zweimal mit je 100 ml Wasser gewaschen, getrocknet, klärfiltriert und kalt gerührt. Dabei kristallisieren 111,4 g farbloses Produkt der Formel 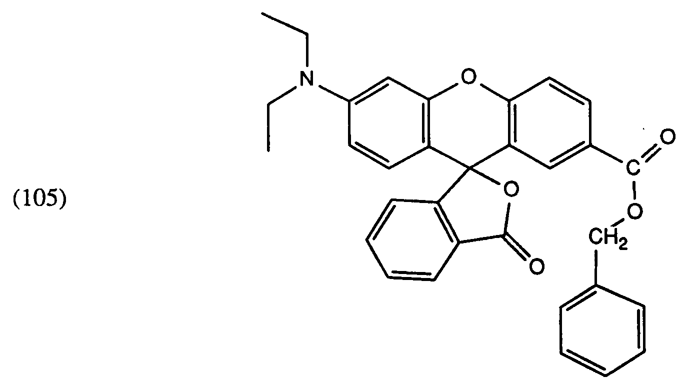 aus.
   Smp.: 108-110°C
   Dieser Farbbildner weist in Kapselölen eine hervorragende Löslichkeit auf, sublimiert nicht und reagiert auf den handelsüblichen CF-Papieren zu einem intensiven Orange.
Herstellungsbeispiel 6: 64 g feuchtes Nutschgut der Formel (103a) werden mit 0,1 g Aliquat 336, 4 ml 10 N Natriumhydroxid-Lösung und 8 ml Propylbromid während einer Stunde bei 20-25°C verrührt. Man gibt nochmals 4 ml Natriumhydroxid-Lösung hinzu und erwärmt unter starkem Rühren auf 80°C. Man hält eine Stunde bei dieser Temperatur, gibt 200 ml Toluol hinzu und trennt die wässrige Phase ab. Die Toluolphase wird nach dreimaligem Waschen mit je 50 ml Wasser eingeengt. Es resultieren 15,4 g Rohprodukt der Formel 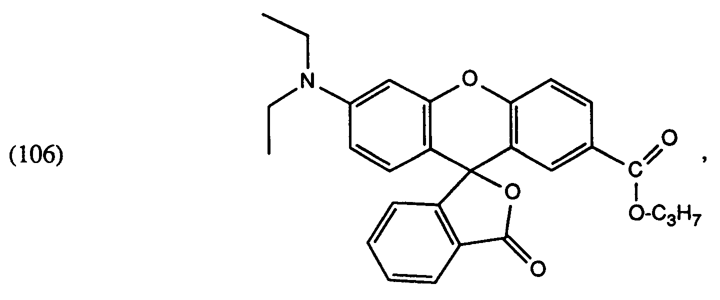 welches nach Umkristallisation aus Isopropylalkohol einen Schmelzpunkt von 110-112°C aufweist. Dieser Farbbildner löst sich hervorragend in Kapselölen, sublimiert nicht und gibt auf CF-Papieren einen orangen Farbton.

Analog zu den erwähnten Herstellungsbeispielen können die Verbindungen der Tabelle 1 hergestellt werden:

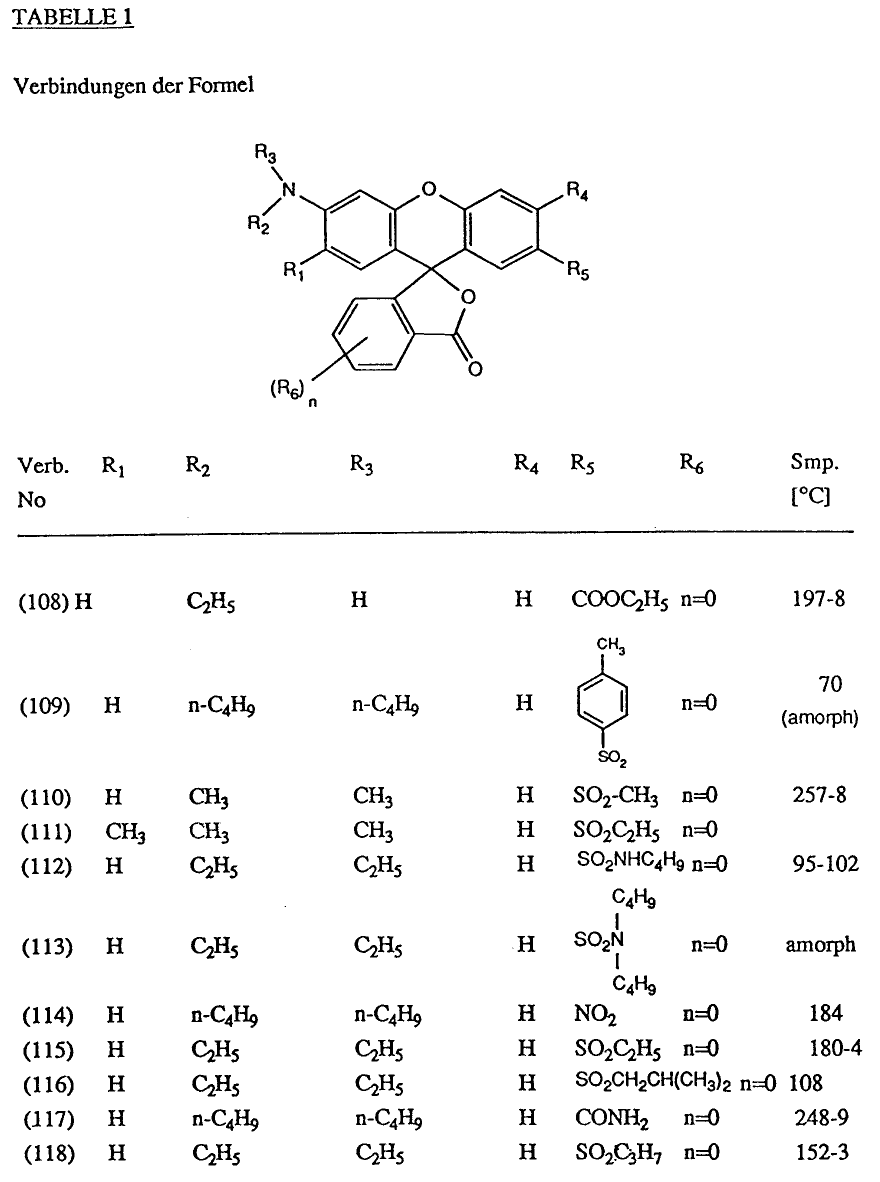

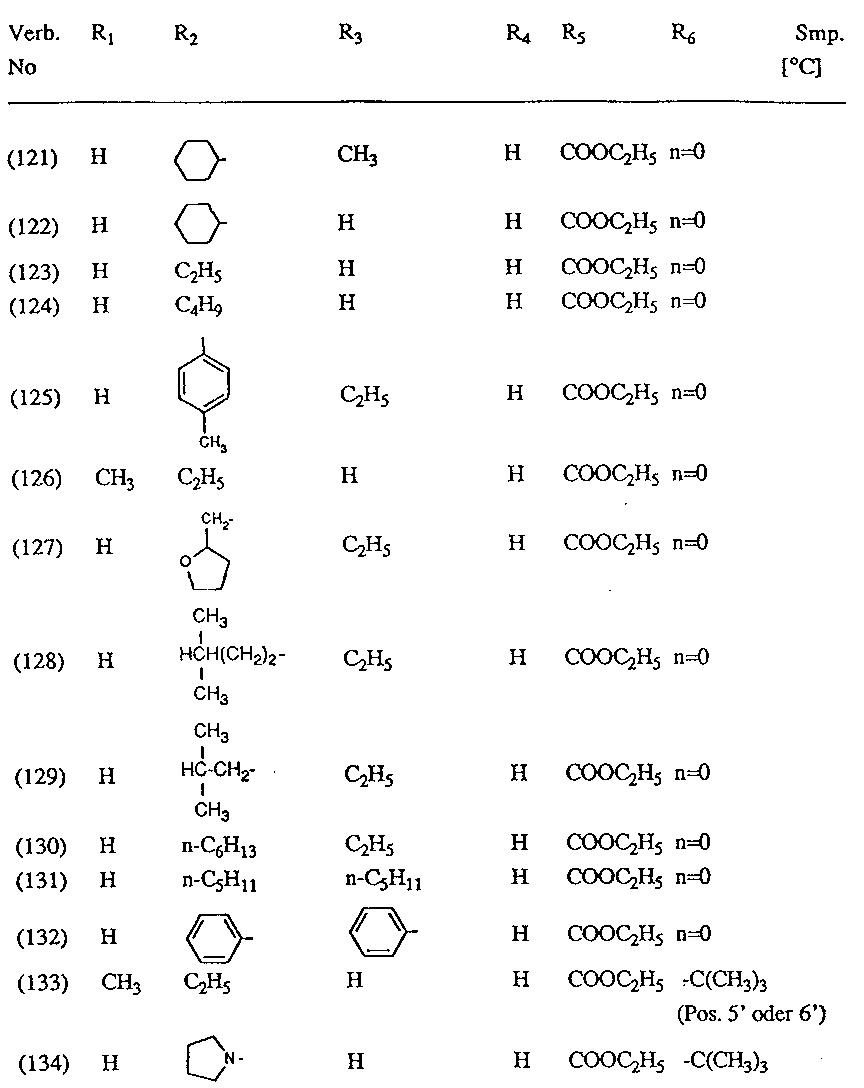

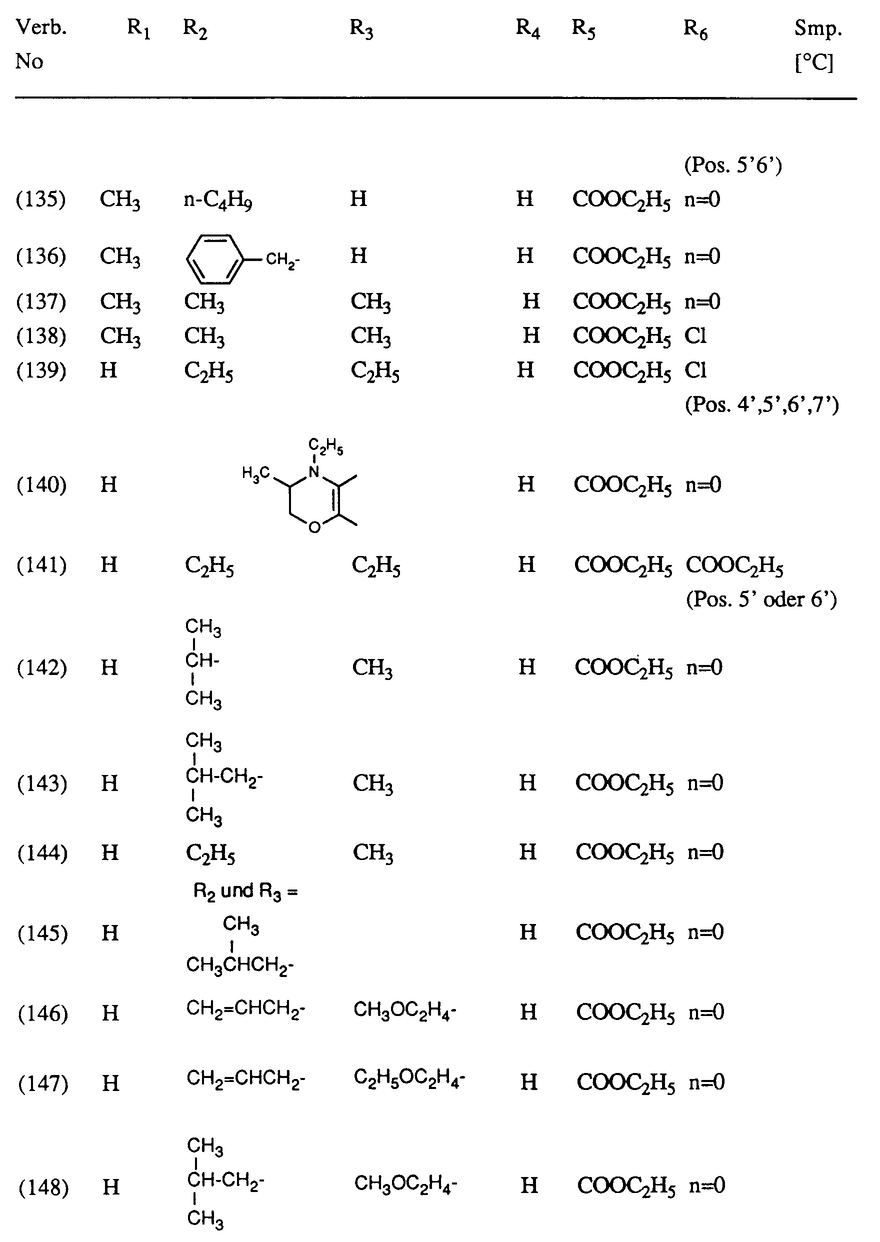

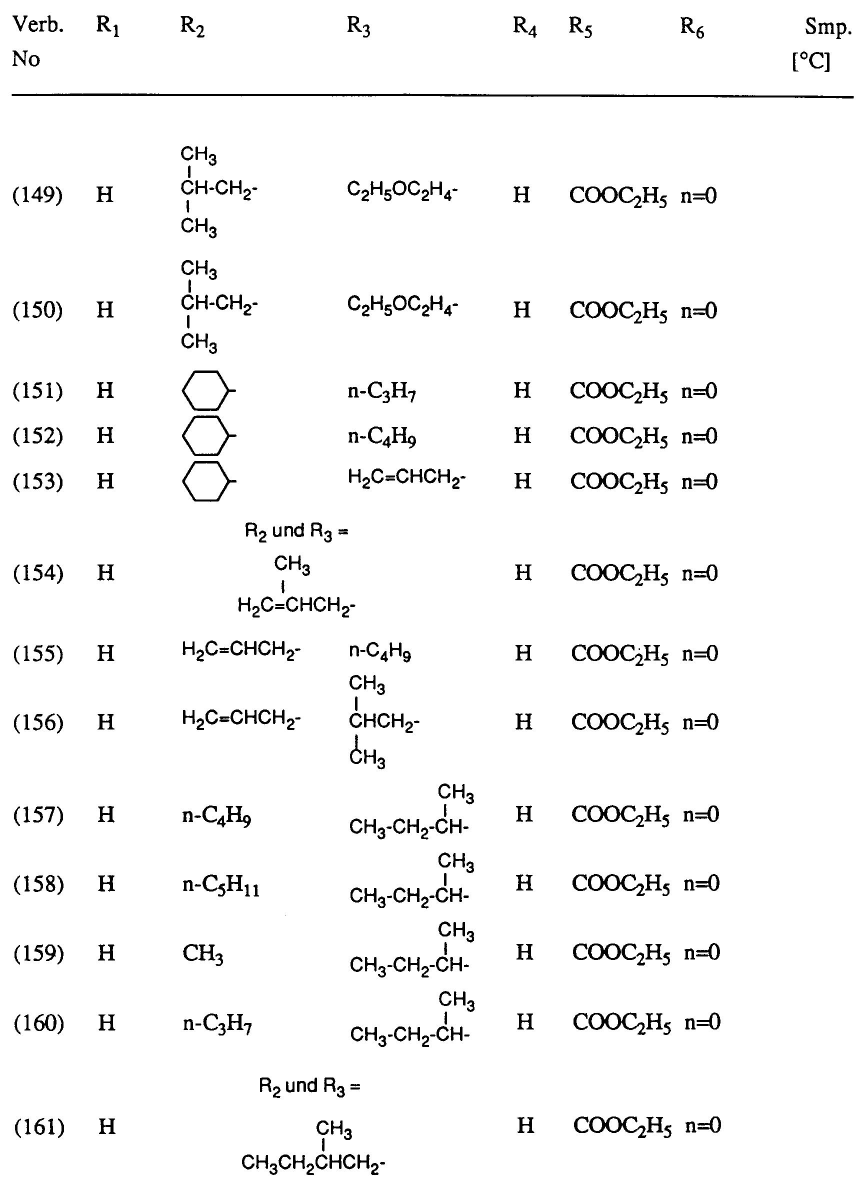

### Anwendungsbeispiel 1: Herstellung eines druckempflindlichem Kopierpapiers

Eine Lösung von 1 g der Fluoranverbindung (101) (Herstellungsbeispiel 1) in 80 g Diisopropylnaphthalin und 19 g Kerosin wird in an sich bekannter Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste, den sublimationsechten Farbbildner enthaltende Blatt und das mit Farbentwickler beschichtete Papier werden mit den Beschichtungen benachbart aufeinandergelegt. Durch Schreiben mit der Hand ,der mit der Schreibmaschine auf dem ersten Blatt wird Druck ausgeübt, und es entwickelt sich sofort auf dem mit dem Entwickler beschichteten Blatt eine intensive orange Kopie, die ausgezeichnet lichtecht ist.

### Anwendungsbeispiel 2: Herstellung von druckempfindlichem Papier für schwarze Kopien

Ersetzt man in Anwendungsbeispiel 1 die Fluoranverbindung der Formel (101) durch eine Mischung aus
- 1 g: der Verbindung (103)
- 0,8 g: 3,3-Bis(4-dimethylaminophenyl)-6-dimethylamino-isobenzofuran-3H-1-on
- 0,8: N-Butylcarbazol-3-yl-bis-(4-N-methyl-N-phenylaminophenyl)-methan
- 2,4 g: 3-Diethylamino-7-dibenzylaminofluoran
und verfährt im übrigen wie in Anwendungsbeispiel 1 beschirieben, so erhält man ein sublimationsechtes, druckempfindliches Aufzeichungsmaterial, welches durch Schreiben mit der Hand oder mit der Schreibmaschine eine intensive und lichtechte schwarze Kopie ergibt.

### Anwendungsbeispiel 3: Sublimations-/Migrationstest

Das nach Anwendungsbeispiel 2 hergestellte schwarzsschreibende Kopierpapier wird auf Sublimations- und Migrationsbeständigkeit wie folgt geprüft:

Die Vorderseite eines mit Aktivton als Farbentwickler beschichteten Blatts (CF-Blatt) wird mit einem Blatt (CB-Blatt), das die mikroverkapselte Formulierung des Anwendungsbeispiels 2 enthaltende schwarzschreibende Mischung enthält, zusammengebracht. Anschliessend wird mit einem Schreibautomaten (Olitex 20) im Zentrum der Prüffläche eine Schriftprobe erzeugt. Danach werden die Ober-und Unterseite mit je 5 (unbehandelten) Basispapieren abgedeckt. Die so erhältlichen Pakete werden mit einer Aluminiumplatte (ca. 1 kg) beschwert und 5 Stunden bei 110° C im Trockenschrank belassen.

Anschliessend wird das Blatt wie folgt begutachtet:
CF-Blatt:
   - Verfärbung
   - Anfärbung (Migration)
Schrift:
   - Nuancenveränderung
   - Intensitätsabnahme
   - Ausbluten

In diesem Test zeigen die geprüften Papiere eine deutliche Verbesserung der Sublimations- bzw. Migrationsechtheit gegenüber bekannten Schwarmischungen.

### Anwendungsbeispiel 4: Herstellung eines wärmeempfindlichen Aufzeichnungsmaterials

In einer Kugelmühle werden 32 g 4,4'-Isopropylidendiphenol (Bisphenol A), 3,8 g Distearylamid des Ethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g der Verbindung (103) gemäss Herstellungsbeispiel 3,3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 g/m² auf ein Papier gestrichen. Durch Berührung des Papiers mit einem erhitzten Metallstift wird eine intensive orange Farbe erhalten, die eine ausgezeichnete Lichtechtheit hat. Das Papier zeigt keine Hintergrundverfärbung.

### Anwendungsbeispiel 5: Herstellung eines wärmeempfindlichen schwarzchreibenden Aufzeichnungsmaterials

Analog zu Anwendungsbeispiel 4 erhält man ein wärmeempfindliches schwarzschreibendes Aufzeichungsmaterial, wenn man anstelle der 6,0 g der Verbindung (103) eine Mischung bestehend aus:
- 3 g: der Verbindung (103) und
- 3 g: 3-Diethylamino-7-dibenzylaminofluoran
verwendet. Das getrocknete Papier entwickelt beim Beschreiben mit einem handelsüblichen Faksimile-Gerät (Infotec 6510) eine schwarze Schrift.

## Patentansprüche

1. Druck- oder wärmeempfindliches Aufzeichungsmaterial enthaltend mindestens einen Farbbildner der Formel 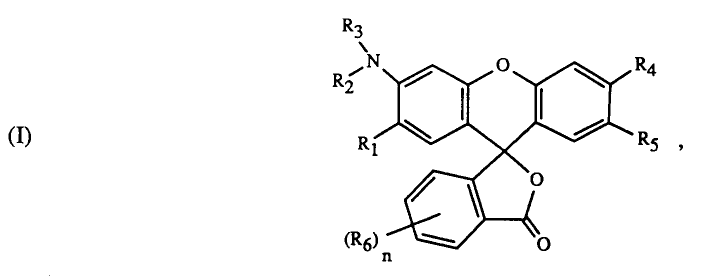 worin
R₁ Wasserstoff oder C₁-C₄-Alkyl;
R₂ und R₃ unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
R₂ und R₃ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring;
R₄ Wasserstoff, Hydroxy oder C₁-C₄-Alkyl;
R₅ Nitro; SO₂R₇; SO₂OR₈; SO₂NR₉R₁₀; COR₁₁; CONR₉R₁₀; oder C₁-C₄-Halogenalkyl; nicht substituierter oder durch Halogen oder Hydroxy substituierter 2-Triazinyl- oder 1-Benztriazolylrest;
R₆ Halogen; Nitro; C₁-C₄-Alkyl; C₁-C₄-Halogenalkyl; Amino; mono-C₁-C₄-Alkylamino; di-C₁-C₄-Alkylamino; oder COR₁₁;
n 0; 1; 2; 3; oder 4;
R₇ C₁-C₈-Alkyl; oder C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-Alkyl;
R₈ Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₄-alkyl;
R₉ und R₁₀ unabhängig voneinander Wasserstoff; oder C₁-C₈-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring; und
R₁₁ Hydroxy; C₁-C₈-Alkyl; C₂-C₈-Alkoxy; C₁-C₈-Halogenalkyl; im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
bedeutet,
mit der Massgabe, dass 3-Diethylamino-7-methylsulfonylfluoran, 3-Diethylamino-7-nitrofluoran, 3-Diethylamino-6-methyl-7-nitrofluoran, 3-Diethylamino-fluoran-7-sulfonsäure, 3-Diethylamino-7-(4-ethoxyphenyl)sulphonylfluoran und 3-Diethylamino-7-benzyloxyfluoran nicht mit umfaßt sind.

2. Aufzeichnungsmaterial nach Anspruch 1 enthaltend mindestens einen Farbbildner der Formel (I), worin
R₁ Wasserstoff oder C₁-C₄-Alkyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten oder durch C₁-C₄-Alkyl substituierten Pyrrolidin- oder Piperidin-Ring;
R₄ Wasserstoff oder C₁-C₄-Alkyl;
R₅ Nitro; SO₂R₇; SO₂OR₈; SO₂NR₉R₁₀; COR₁₁; CONR₉R₁₀; oder C₁-C₄-Halogenalkyl;
n 0, 1, 2, 3 oder 4;
R₆ wenn n 1,2,3 oder 4 ist, Halogen; wenn n 1 oder 2 ist, C₁-C₄-Alkyl, C₁-C₄-Halogenalkyl; oder wenn n 1 ist, Nitro, COR₁₁, Amino, mono-C₁-C₄-Alkylamino oder di-C₁-C₄Alkylamino;
R₇ C₁-C₄-Alkyl; oder C₁-C₄-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₂-alkyl;
R₈ Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
R₉ und R₁₀ unabhängig voneinander Wasserstoff; oder C₁-C₄-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin- oder Piperidin-Ring; und
R₁₁ C₁-C₄-Alkyl; C₁-C₄-Halogenalkyl; C₂-C₄-Alkoxy; im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy
bedeutet.

3. Aufzeichungsmaterial nach Anspruch 1 oder 2 enthaltend mindestens einen Farbbildner der Formel (I), worin
R₁ Wasserstoff oder Methyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten Pyrrolidin- oder Piperidin-Ring;
R₄ Wasserstoff oder Methyl;
R₅ Nitro; SO₂R₇; SO₂NR₉R₁₀; COR₁₁; CONR₉R₁₀; oder C₁-C₄-Halogenalkyl;
n 0, 1, 2, 3 oder 4;
R₆ wenn n 1,2,3 oder 4 ist, Halogen; wenn n 1 oder 2 ist, Methyl; oder wenn n 1 ist, Nitro, Amino, mono-C₁-C₄-Alkylamino oder di-C₁-C₄-Alkylamino;
R₇ C₁-C₄-Alkyl; oder C₁-C₄-Halogenalkyl; unsubstituiertes oder im Phenylrest durch Halogen , C₁-C₄-Alkyl, oder C₁-C₄-Alkoxy, substituiertes Phenyl;
R₉ und R₁₀ unabhängig voneinander Wasserstoff; oder C₁-C₄-Alkyl;
R₁₁ C₁-C₄-Alkyl; C₂-C₄-Alkoxy; im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder Phenyl-C₁-C₂-Alkoxy
bedeutet.

4. Aufzeichungsmaterial nach einem der Ansprüche 1 bis 3 enthaltend mindestens einen Farbbildner der Formel (I), worin
R₅ SO₂R₇ bedeutet.

5. Aufzeichungsmaterial nach einem der Ansprüche 1 bis 3 enthaltend mindestens einen Farbbildner der Formel (I), worin
R₅ SO₂OR₈ bedeutet.

6. Aufzeichungsmaterial nach einem der Ansprüche 1 bis 3 enthaltend mindestens einen Farbbildner der Formel (I), worin
R₅ SO₂NR₉R₁₀ bedeutet.

7. Aufzeichungsmaterial nach einem der Ansprüche 1 bis 3 enthaltend mindestens einen Farbbildner der Formel (I), worin
R₅ COR₁₁ bedeutet.

8. Aufzeichnungsmaterial nach Anspruch 7, dadurch gekennzeichnet, dass
R₁₁ C₁-C₄-Alkyl, C₂-C₄-Alkoxy oder Phenyl-C₁-C₂-Alkoxy bedeutet.

9. Aufzeichnungsmaterial nach Anspruch 7 oder 8, dadurch gekennzeichnet, dass
R₁₁ C₂-C₄-Alkoxy oder Phenyl-C₁-C₂-Alkoxy bedeutet.

10. Aufzeichungsmaterial nach einem der Ansprüche 1 bis 3 enthaltend mindestens einen Farbbildner der Formel (I), worinR₅ CONR₉R₁₀ bedeutet.

11. Aufzeichungsmaterial nach einem der Ansprüche 1 bis 3 enthaltend mindestens einen Farbbildner der Formel (I), worinR₅ C₁-C₄-Halogenalkyl bedeutet.

12. Aufzeichungsmaterial nach einem der Ansprüche 1 bis 3 enthaltend mindestens einen Farbbildner der Formel (I), worin R₅ Nitro bedeutet.

13. Fluorane der allgemeinen Formel 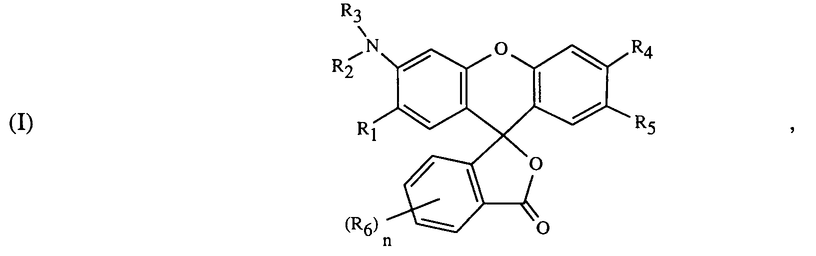 worin
R₁ Wasserstoff oder C₁-C₄-Alkyl;
R₂ und R₃ unabhängig voneinander Wasserstoff; C₁-C₈-Alkyl; nicht substituiertes oder durch C₁-C₄-Alkyl oder Halogen substituiertes C₄-C₇-Cycloalkyl; nicht substituiertes oder durch C₁-C₄-Alkyl, Hydroxy oder Halogen substituiertes Phenyl; Phenyl-C₁-C₄-Alkyl; C₃-C₆-Alkenyl; C₁-C₄-Alkoxy; C₁-C₄-Alkoxy-C₁-C₄Alkyl; 2-Tetrahydrofuranyl, oder
R₂ und R₃ gemeinsam mit dem Stickstoffatom, an welches sie gebunden sind, einen unsubstituienen oder durch C₁-C₄-Alkyl substituierten Pyrrolidin-, Piperidin-, Morpholin-, Thiomorpholin- oder Piperazin-Ring;
R₄ Wasserstoff, Hydroxy oder C₁-C₄-Alkyl;
R₅ Nitro; SO₂R₇; SO₂OR₈; SO₂NR₉R₁₀; COR₁₁; CONR₉R₁₀; oder C₁-C₄-Halogenalkyl; nicht substituierter oder durch Halogen oder Hydroxy substituierter 2-Triazinyl- oder 1-Benztriazolylrest;
R₆ Halogen; Nitro; C₁-C₄-Alkyl; C₁-C₄-Halogenalkyl; Amino; mono-C₁-C₄-Alkylamino; di-C₁-C₄-Alkylamino; oder COR₁₁;
n 0; 1; 2; 3; oder 4;
R₇ C₁-C₈-Alkyl; oder C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₄-Alkyl;
R₈ Wasserstoff; C₁-C₈-Alkyl; C₁-C₈-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₄-alkyl;
R₉ und R₁₀ unabhängig voneinander Wasserstoff; oder C₁-C₈-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring; und
R₁₁ Hydroxy; C₁-C₈-Alkyl; C₂-C₈-Alkoxy; C₁-C₈-Halogenalkyl; im Phenylrest durch Halogen, C₁-C₄-Alkyl, C₁-C₄-Halogenalkyl, C₁-C₄-Alkoxy substituiertes Phenyl; Phenyl-C₁-C₄-Alkyl oder Phenyl-C₁-C₄-Alkoxy;
bedeutet, mit der Massgabe, dass 3-Diethylamino-7-methylsulfonylfluoran, 3-Diethylamino-7-nitrofluoran, 3-Diethylamino-6-methyl-7-nitrofluoran, und 3-Diethylamino-fluoran-7-sulfonsäure nicht mit umfaßt sind.

14. Fluorane gemäss Anspruch 13
worin
R₁ Wasserstoff oder C₁-C₄-Alkyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten oder durch C₁-C₄-Alkyl substituierten Pyrrolidin- oder Piperidin-Ring;
R₄ Wasserstoff oder C₁-C₄-Alkyl;
R₅ Nitro; SO₂R₇; SO₂OR₈; SO₂NR₉R₁₀; COR₁₁; CONR₉R₁₀; oder C₁-C₄-Halogenalkyl;
n 0, 1, 2, 3 oder 4;
R₆ wenn n 1, 2, 3 oder 4 ist, Halogen; wenn n 1 oder 2 ist, C₁-C₄-Alkyl, C₁-C₄-Halogenalkyl; oder wenn n 1 ist, Nitro, COR₁₁, Amino, mono-C₁-C₄-Alkylamino oder di-C₁-C₄Alkylamino;
R₇ C₁-C₄-Alkyl; oder C₁-C₄-Halogenalkyl; unsubstituiertes oder im Phenylrest durch Halogen, C₁-C₄-Alkyl, C₁-C₄-Halogenalkyl, C₁-C₄-Alkoxy substituiertes Phenyl oder Phenyl-C₁-C₂-alkyl;
R₈ Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
R₉ und R₁₀ unabhängig voneinander Wasserstoff; oder C₁-C₄-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin- oder Piperidin-Ring; und
R₁₁ C₁-C₄-Alkyl; C₁-C₄-Halogenalkyl; C₂-C₄-Alkoxy; im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy
bedeutet.

15. Fluorane gemäss Anspruch 13 oder 14,
worin
R₁ Wasserstoff oder C₁-C₄-Alkyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten oder durch C₁-C₄-Alkyl substituierten Pyrrolidin- oder Piperidin-Ring;
R₄ Wasserstoff oder C₁-C₄-Alkyl;
R₅ Nitro; SO₂R₇; SO₂OR₈; SO₂NR₉R₁₀; COR₁₁; CONR₉R₁₀; oder C₁-C₄-Halogenalkyl;
n 0, 1, 2, 3 oder 4;
R₆ wenn n 1, 2, 3 oder 4 ist, Halogen; wenn n 1 oder 2 ist, Nitro, C₁-C₄-Alkyl, C₁-C₄-Halogenalkyl; oder wenn n 1 ist, COR₁₁, Amino, Mono-C₁-C₄-Alkylamino oder Di-C₁-C₄Alkylamino;
R₇ C₁-C₄-Alkyl; oder C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
R₈ Wasserstoff; C₁-C₄-Alkyl; C₁-C₄-Halogenalkyl; unsubstituiertes oder im Phenylrest durch Halogen, C₁-C₄-Alkyl, C₁-C₄-Halogenalkyl, C₁-C₄-Alkoxy, substituiertes Phenyl oder Phenyl-C₁-C₂-alkyl;
R₉ und R₁₀ unabhängig voneinander Wasserstoff; oder C₁-C₄-Alkyl; oder
R₉ und R₁₀ gemeinsam mit dem Stickstoffatom, an welches sie gebunden sind, einen unsubstituierten oder durch C₁-C₄-Alkyl substituierten Pyrrolidin- oder Piperidin-Ring; und
R₁₁ C₁-C₄-Alkyl; C₁-C₄-Halogenalkyl; C₂-C₄-Alkoxy; im Phenylrest durch Halogen, C₁-C₄-Alkyl, C₁-C₄-Halogenalkyl, C₁-C₄-Alkoxy, substituiertes Phenyl; Phenyl-C₁-C₂-Alkyl oder Phenyl-C₁-C₂-Alkoxy;
bedeutet.

16. Fluorane gemäss einem der Ansprüche 13 bis 15,
worin
R₁ Wasserstoff oder Methyl;
R₂ und R₃ unabhängig voneinander Wasserstoff; C₁-C₅-Alkyl; oder
R₂ und R₃ gemeinsam mit dem Stickstoffatom, an welches sie gebunden sind, einen unsubstituierten Pyrrolidin- oder Piperidin-Ring;
R₄ Wasserstoff oder Methyl;
R₅ Nitro; SO₂R₇; SO₂NR₉R₁₀; COR₁₁; CONR₉R₁₀; oder C₁-C₄-Halogenalkyl;
n 0, 1, 2, 3 oder 4;
R₆ wenn n 1, 2, 3 oder 4 ist, Halogen; wenn n 1 oder 2 ist, Nitro, Methyl; oder wenn n 1 ist, Nitro, Amino, Mono-C₁-C₄-Alkylamino oder Di-C₁-C₄-Alkylamino;
R₇ C₁-C₄-Alkyl; oder C₁-C₄-Halogenalkyl; unsubstituiertes oder im Phenylrest durch Halogen, C₁-C₄-Alkyl, oder C₁-C₄-Alkoxy substituiertes Phenyl;
R₉ und R₁₀ unabhängig voneinander Wasserstoff oder C₁-C₄-Alkyl;
R₁₁ C₁-C₄-Alkyl; C₂-C₄-Alkoxy; im Phenylrest durch Halogen, C₁-C₄-Alkyl, C₁-C₄-Halogenalkyl oder C₁-C₄-Alkoxy substituiertes Phenyl; Phenyl-C₁-C₂-Alkyl oder C₁-C₂-Alkoxy
bedeutet.

17. Fluorane gemäss einem der Ansprüche 13 bis 16, worin R₅ SO₂R₇ bedeutet.

18. Fluorane gemäss einem der Ansprüche 13 bis 16, worin R₅ SO₂OR₈ bedeutet.

19. Fluorane gemäss einem der Ansprüche 13 bis 16, worin R₅ SO₂NR₉R₁₀ bedeutet.

20. Fluorane gemäss einem der Ansprüche 13 bis 16, worin R₅ COR₁₁ bedeutet.

21. Fluorane nach Anspruch 20, dadurch gekennzeichnet, dass R₁₁ C₁-C₄-Alkyl, C₂-C₄-Alkoxy oder Phenyl-C₁-C₂-Alkoxy bedeutet.

22. Fluorane nach Anspruch 20 oder 21, dadurch gekennzeichnet, dass R₁₁ C₂-C₄Alkoxy oder Phenyl-C₁-C₂-Alkoxy bedeutet.

23. Fluorane gemäss einem der Ansprüche 13 bis 16, worin R₅ CONR₉R₁₀ bedeutet.

24. Fluorane gemäss einem der Ansprüche 13 bis 16, worin R₅ C₁-C₄-Halogenalkyl bedeutet.

25. Fluorane gemäss einem der Ansprüche 13 bis 16, worin
R₅ Nitro und
R₂ und R₃ unabhängig voneinander Wasserstoff oder C₄-C₈-Alkyl;
R₂ und R₃ gemeinsam mit dem Stickstoffatom, an welches sie gebunden sind, einen unsubstituierten oder durch C₁-C₄-Alkyl substituierten Pyrrolidin-, Piperidin-, Morpholin-, Thiomorpholin- oder Piperazin-Ring bedeutet.

26. Verfahren zur Herstellung von Verbindungen der Formel I, nach einem der Ansprüche 13 bis 25, gekennzeichnet durch die Umsetzung eines Benzophenons der Formel II mit einem Phenol oder Phenolether der Formel (III) 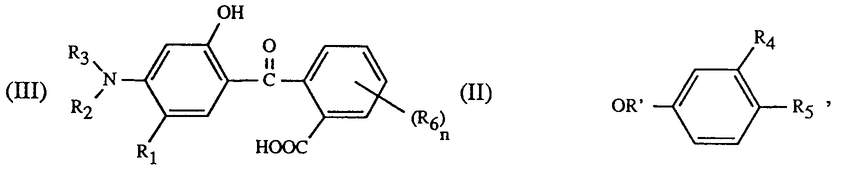 worin die Reste R₁ bis R₆ und n wie zuvor definiert sind und R' Wasserstoff oder C₁-C₄-Alkyl bedeutet, bei Temperaturen von vorzugsweise 0 bis 70° C, in 50 bis 100 %iger Schwefelsäure zu einem Phthalid der Formel 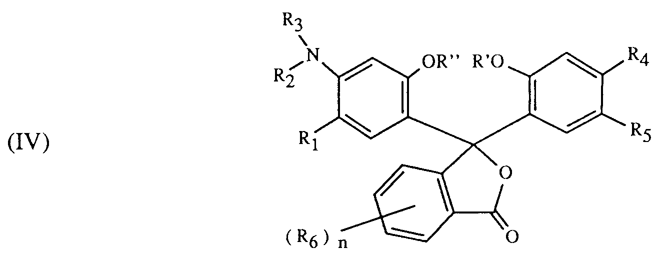 kondensiert und anschliessender Cyclisierung bei Temperaturen von 20 bis 100° C zu einer Verbindung der Formel I.

27. Verfahren zur Herstellung eines Sulfons der Formel I nach Anspruch 17, durch Umsetzung eines Sulfonesters der Formel I nach Anspruch 18 mit einem Carbanion oder Grignard-Reagenz der allgemeinen Formel
R₇⁻M⁺ oder R₇Mg-Hal, (V)
worin
M⁺ ein Kationenäquivalent eines Alkali- oder Erdalkalimetallions; und
Hal Halogen
bedeutet und R₇ wie zuvor definiert ist.

28. Verfahren zur Herstellung eines Sulfonamids der Formel I nach Anspruch 19, durch Umsetzung eines Sulfonesters der Formel I nach Anspruch 18 mit einem Amin der Formel
HNR₉R₁₀, (VI)
worin R₉ und R₁₀ wie zuvor definiert sind.

29. Verfahren zur Herstellung mikroverkapselter Farbbildner, dadurch gekennzeichnet, dass man mindestens eine Verbindung der Formel I nach einem oder mehreren der Ansprüche 1 bis 12 mikroverkapselt.

30. Verfahren zur Herstellung mikroverkapselter Farbbildner, dadurch gekennzeichnet, dass man mindestens eine Verbindung der Formel I nach einem oder mehreren der Ansprüche 13 bis 25 mikroverkapselt.

31. Verfahren zur Herstellung eines druckempfindlichen Aufzeichungsmaterials, dadurch gekennzeichnet, dass man auf einen ersten Träger Mikrokapseln enthaltend mindestens eine Verbindung der Formel I nach einem der Ansprüche 1 bis 12 aufträgt und auf den ersten Träger oder auf einen zweiten Träger, der in unmittelbaren Kontakt zu dem ersten Träger angeordnet ist, einen Entwickler aufträgt.

32. Verfahren zur Herstellung eines druckempfindlichen Aufzeichungsmaterials, dadurch gekennzeichnet, dass man auf einen ersten Träger Mikrokapseln enthaltend eine Verbindung der Formel I nach einem der Ansprüche 13 bis 25 aufträgt und auf den ersten Träger oder auf einen zweiten Träger, der in unmittelbarem Kontakt zu dem ersten Träger angeordnet ist, einen Entwickler aufträgt.

33. Verfahren zur Herstellung eines wärmeempfindlichen Aufzeichungsmaterials, dadurch gekennzeichnet, dass man auf einen Träger eine Misching von Farbbildnern enthaltend mindestens eine Verbindung der Formel I nach einem der Ansprüche 1 bis 12 und einen Entwickler aufträgt.

34. Verfahren zur Herstellung eines wärmeempfindlichen Aufzeichungsmaterials, dadurch gekennzeichnet, dass man auf einen Träger eine Mischung von Farbbildnern enthaltend eine Verbindung der Formel I nach einem der Ansprüche 13 bis 25 und einen Entwickler aufträgt.

35. Druck- oder wärmeempfindliches Aufzeichungsmaterial, enthaltend eine Mischung von Farbbildnern mit mindestens einer Verbindung der Formel I nach einem der Ansprüche 1 bis 12 und einen Entwickler.

36. Druck- oder wärmeempfindliches Aufzeichungsmaterial, enthaltend eine Mischung von Farbbildnern mit mindestens einer Verbindung der Formel I nach einem der Ansprüche 13 bis 25 und einen Entwickler.

## Claims

1. A pressure-sensitive or heat-sensitive recording material comprising at least one colour former of the formula 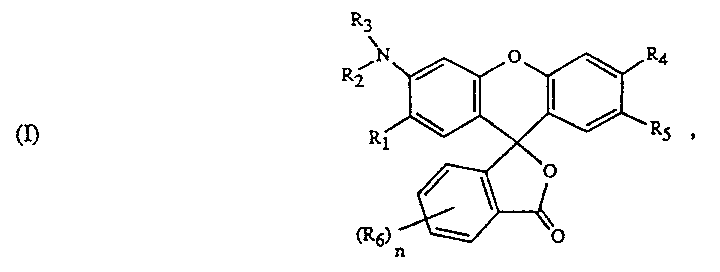 wherein
R₁ is hydrogen or C₁-C₄alkyl;
R₂ and R₃ are each independently of the other hydrogen; C₁-C₈alkyl; unsubstituted or C₁-C₄alkyl- or halogen-substituted C₄-C₇cycloalkyl; unsubstituted phenyl or phenyl which is substituted by C₁-C₄alkyl, hydroxy or halogen; phenyl-C₁-C₄alkyl; C₃-C₆alkenyl; C₁-C₄alkoxy; C₁-C₄alkoxy-C₁-C₄alkyl; 2-tetrahydrofuranyl, or
R₂ and R₃ together with the linking nitrogen atom are an unsubstituted or C₁-C₄alkyl-substituted pyrrolidine, piperidine, morpholine, thiomorpholine or piperazine ring;
R₄ is hydrogen, hydroxy or C₁-C₄alkyl;
R₅ is nitro; SO₂R₇; SO₂OR₈; SO₂NR₉R₁₀; CDR₁₁; CONR₉R₁₀; or C₁-C₄haloalkyl; an unsubstituted or a halogen- or hydroxy-substituted 2-triazinyl or 1-benzotriazolyl radical;
R₆ is halogen; nitro; C₁-C₄alkyl; C₁-C₄haloalkyl; amino; mono-C₁-C₄alkylamino; di-C₁-C₄alkylamino; or COR₁₁;
n is 0; 1; 2; 3; or 4;
R₇ is C₁-C₈alkyl; or C₁-C₈haloalkyl; unsubstituted phenyl or phenyl-C₁-C₄alkyl; or phenyl or phenyl-C₁-C₄alkyl which is substituted in the phenyl radical by halogen, C₁-C₄alkyl, C₁-C₄haloalkyl or C₁-C₄alkoxy;
R₈ is hydrogen; C₁-C₈alkyl; C₁-C₈haloalkyl; unsubstituted phenyl or phenyl-C₁-C₄alkyl; or phenyl or phenyl-C₁-C₄alkyl which is substituted in the phenyl radical by halogen, C₁-C₄alkyl, C₁-C₄haloalkyl or C₁-C₄alkoxy;
R₉ and R₁₀ are each independently of the other hydrogen; or C₁-C₈alkyl; or
R₉ and R₁₀ together with the linking nitrogen atom are an unsubstituted or a C₁-C₄alkyl-substituted pyrrolidine, piperidine, morpholine, thiomorpholine or piperazine ring; and
R₁₁ is hydroxy; C₁-C₈alkyl; C₂-C₈alkoxy; C₁-C₈haloalkyl; phenyl which is substituted by halogen, C₁-C₄alkyl, C₁-C₄haloalkyl or C₁-C₄alkoxy; phenyl-C₁-C₄alkyl or phenyl C₁-C₄alkoxy;
with the provision that 3-diethylamino-7-methylsulfonylfluoran, 3-diethylamino-7-nitrofluoran, 3-diethylamino-6-methyl-7-nitrofluoran, 3-diethylaminofluoran-7-sulfonic acid, 3-diethylamino-7-(4-ethoxyphenyl)sulfonylfluoran and 3-diethylamino-7-benzyloxyfluoran are not comprised.

2. A recording material according to claim 1, comprising at least one colour former of the formula (I) wherein
R₁ is hydrogen or C₁-C₄alkyl;
R₂ and R₃ are each independently of the other hydrogen; C₁-C₅alkyl; or
R₂ and R₃ together with the linking nitrogen atom are an unsubstituted or C₁-C₄alkyl-substituted pyrrolidine or piperidine ring;
R₄ is hydrogen or C₁-C₄alkyl;
R₅ is nitro; SO₂R₇; SO₂OR₈; SO₂NR₉R₁₀; COR₁₁; CONR₉R₁₀; or C₁-C₄haloalkyl;
n is 0, 1, 2, 3 or 4;
R₆ is halogen when n is 1, 2, 3 or 4; C₁-C₄alkyl or C₁-C₄haloalkyl when n is 1 or 2; or, when n is 1, nitro, COR₁₁, amino, mono-C₁-C₄alkylamino or di-C₁-C₄alkylamino;
R₇ is C₁-C₄alkyl; C₁-C₄haloalkyl; unsubstituted phenyl or phenyl-C₁-C₂alkyl; or phenyl or phenyl-C₁-C₂alkyl which is substituted in the phenyl radical by halogen, C₁-C₄alkyl, C₁-C₄haloalkyl or C₁-C₄alkoxy;
R₈ is hydrogen, C₁-C₄alkyl; C₁-C₄haloalkyl; unsubstituted phenyl or phenyl-C₁-C₂alkyl; or phenyl or phenyl-C₁-C₂alkyl which is substituted in the phenyl radical by halogen, C₁-C₄alkyl, C₁-C₄haloalkyl or C₁-C₄alkoxy;
R₉ and R₁₀ are each independently of the other hydrogen; or C₁-C₄alkyl; or
R₉ and R₁₀ together with the linking nitrogen atom are an unsubstituted or a C₁-C₄alkyl-substituted pyrrolidine or piperidine ring; and
R₁₁ is C₁-C₄alkyl; C₁-C₄haloalkyl; C₂-C₄alkoxy; phenyl which is substituted by halogen, C₁-C₄alkyl, C₁-C₄haloalkyl or C₁-C₄alkoxy; phenyl-C₁-C₂alkyl or phenyl-C₁-C₂alkyl or phenyl-C₁-C₂alkoxy.

3. A recording material according to claim 1 or 2 comprising at least one colour former of the formula wherein
R₁ is hydrogen or methyl;
R₂ and R₃ are each independently of the other hydrogen; C₁-C₅alkyl; or
R₂ and R₃ together with the linking nitrogen atom are an unsubstituted pyrrolidine or piperidine ring;
R₄ is hydrogen or methyl;
R₅ is nitro; SO₂R₇; SO₂NR₉R₁₀; COR₁₁; CONR₉R₁₀; or C₁-C₄-haloalkyl;
n is 0, 1, 2, 3 or 4;
R₆ is halogen when n is 1, 2, 3 or 4; or is methyl when n is 1 or 2; or, when n is 1, is nitro, amino, mono-C₁-C₄alkylamino or di-C₁-C₄alkylamino;
R₇ is C₁-C₄alkyl; or C₁-C₄haloalkyl; unsubstituted phenyl or phenyl which is substituted by halogen, C₁-C₄alkyl or C₁-C₄alkoxy;
R₉ and R₁₀ are each independently of the other hydrogen; or C₁-C₄alkyl;
R₁₁ is C₁-C₄alkyl; C₂-C₄alkoxy; phenyl which is substituted by halogen, C₁-C₄alkyl, C₁-C₄haloalkyl or C₁-C₄alkoxy; phenyl-C₁-C₂alkyl or phenyl-C₁-C₂alkoxy.

4. A recording material according to any one of claims 1 to 3 comprising at least one colour former of formula (I) wherein R₅ is SO₂R₇.

5. A recording material according to any one of claims 1 to 3 comprising at least one colour former of the formula (I) wherein R₅ is SO₂R₈.

6. A recording material according to any one of claims 1 to 3 comprising at least one colour former of the formula (I) wherein R₅ is SO₂NR₉R₁₀.

7. A recording material according to any one of claims 1 to 3 comprising at least one colour former of the formula (I) wherein R₅ is COR₁₁.

8. A recording material according to claim 7 wherein R₁₁ is C₁-C₄alkyl, C₂-C₄alkoxy or phenyl-C₁-C₂alkoxy.

9. A recording material according to claim 7 or claim 8, wherein R₁₁ is C₂-C₄alkoxy or phenyl-C₁-C₂alkoxy.

10. A recording material according to any one of claims 1 to 3 comprising at least one colour former of the formula (I) wherein R₅ is CONR₉R₁₀.

11. A recording material according to any one of claims 1 to 3 comprising at least one colour former of the formula (I) wherein R₅ is C₁-C₄haloalkyl.

12. A recording material according to any one of claims 1 to 3 comprising at least one colour former of the formula (I) wherein R₅ is nitro.

13. A fluoran of the general formula 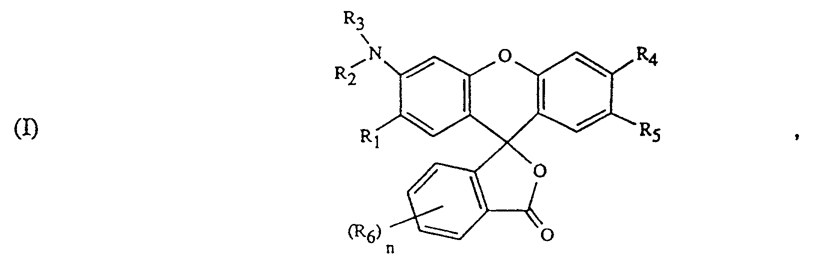 wherein
R₁ is hydrogen or C₁-C₄alkyl;
R₂ and R₃ are each independently of the other hydrogen; C₁-C₈alkyl; unsubstituted or C₁-C₄alkyl- or halogen substituted C₄-C₇cycloalkyl; unsubstituted phenyl or phenyl which is substituted by C₁-C₄alkyl, hydroxy or halogen; phenyl-C₁-C₄alkyl; C₃-C₆alkenyl; C₁-C₄alkoxy; C₁-C₄alkoxy-C₁-C₄alkyl; 2-tetrahydrofuranyl, or
R₂ and R₃ together with the linking nitrogen atom are an unsubstituted or C₁-C₄alkyl-substituted pyrrolidine, piperidine, morpholine, thiomorpholine or piperazine ring;
R₄ is hydrogen, hydroxy or C₁-C₄alkyl;
R₅ is nitro; SO₂R₇; SO₂OR₈; SO₂NR₉R₁₀; COR₁₁; CONR₉R₁₀; or C₁-C₄haloalkyl; an unsubstituted or a halogen- or hydroxy-substituted 2-triazinyl or 1-benzotriazolyl radical;
R₆ is halogen; nitro; C₁-C₄alkyl; C₁-C₄haloalkyl; amino; mono-C₁-C₄alkylamino; di-C₁-C₄alkylamino; or COR₁₁;
n is 0; 1; 2; 3; or 4;
R₇ is C₁-C₈alkyl; or C₁-C₈haloalkyl; unsubstituted phenyl or phenyl-C₁-C₄alkyl; or phenyl or phenyl-C₁-C₄alkyl which is substituted in the phenyl radical by halogen, C₁-C₄alkyl, C₁-C₄haloalkyl or C₁-C₄alkoxy;
R₈ is hydrogen; C₁-C₈alkyl; C₁-C₈haloalkyl; unsubstituted phenyl or phenyl-C₁-C₄alkyl; or phenyl or phenyl-C₁-C₄alkyl which is substituted in the phenyl radical by halogen, C₁-C₄alkyl, C₁-C₄haloalkyl or C₁-C₄alkoxy;
R₉ and R₁₀ are each independently of the other hydrogen; or C₁-C₈alkyl; or
R₉ and R₁₀ together with the linking nitrogen atom are an unsubstituted or a C₁-C₄alkyl-substituted pyrrolidine, piperidine, morpholine, thiomorpholine or piperazine ring; and
R₁₁ is hydroxy; C₁-C₈alkyl; C₂-C₈alkoxy; C₁-C₈haloalkyl; phenyl which is substituted by halogen, C₁-C₄alkyl, C₁-C₄haloalkyl or C₁-C₄alkoxy; or phenyl-C₁-C₄alkyl or phenyl-C₁-C₄alkoxy;
with the proviso that 3-diethylamino-7-methylsulfonylfluoran, 3-diethylamino-7-nitrofluoran, 3-diethylamino-6-methyl-7-nitrofluoran, and 3-diethylaminofluoran-7-sulfonic acid are not comprised.

14. A fluoran according to claim 13, wherein
R₁ is hydrogen or C₁-C₄alkyl;
R₂ and R₃ are each independently of the other hydrogen; C₁-C₅alkyl; or
R₂ and R₃ together with the linking nitrogen atom are an unsubstituted or C₁-C₄alkyl-substituted pyrrolidine or piperidine ring;
R₄ is hydrogen or C₁-C₄alkyl;
R₅ is nitro; SO₂R₇; SO₂OR₈; SO₂NR₉R₁₀; COR₁₁; CONR₉R₁₀; or C₁-C₄haloalkyl;
n is 0, 1, 2, 3 or 4;
R₆ is halogen when n is 1, 2, 3 or 4; or is C₁-C₄alkyl or C₁-C₄haloalkyl when n is 1 or 2; or, when n is 1, is nitro, COR₁₁, amino, mono-C₁-C₄alkylamino or di-C₁-C₄alkylamino;
R₇ is C₁-C₄alkyl; or C₁-C₄haloalkyl; unsubstituted phenyl or phenyl-C₁-C₂alkyl; or phenyl or phenyl-C₁-C₂alkyl which is substituted in the phenyl radical by halogen, C₁-C₄alkyl, C₁-C₄haloalkyl or C₁-C₄alkoxy;
R₈ is hydrogen, C₁-C₄alkyl; C₁-C₄haloalkyl; unsubstituted phenyl or phenyl-C₁-C₂-alkyl; or phenyl or phenyl-C₁-C₂alkyl which is substituted in the phenyl radical by halogen, C₁-C₄alkyl, C₁-C₄haloalkyl or C₁-C₄alkoxy;
R₉ and R₁₀ are each independently of the other hydrogen; or C₁-C₄alkyl; or
R₉ and R₁₀ together with the linking nitrogen atom are an unsubstituted or a C₁-C₄alkyl-substituted pyrrolidine or piperidine ring; and
R₁₁ is C₁-C₄alkyl; C₁-C₄haloalkyl; C₂-C₄alkoxy; phenyl which is substituted by halogen, C₁-C₄alkyl, C₁-C₄haloalkyl or C₁-C₄alkoxy; phenyl-C₁-C₂alkyl or phenyl-C₂-C₂alkoxy.

15. A fluoran according to claim 13 or 14, wherein
R₁ is hydrogen or C₁-C₄alkyl;
R₂ and R₃ are each independently of the other hydrogen; C₁-C₅alkyl; or
R₂ and R₃ together with the linking nitrogen atom are an unsubstituted or C₁-C₄alkyl-substituted pyrrolidine or piperidine ring;
R₄ is hydrogen or C₁-C₄alkyl;
R₅ is nitro; SO₂R₇; SO₂OR₈; SO₂NR₉R₁₀; COR₁₁; CONR₉R₁₀; or C₁-C₄-haloalkyl;
n is 0, 1, 2, 3 or 4;
R₆ is halogen when n is 1, 2, 3 or 4; or is nitro, C₁-C₄alkyl, C₁-C₄haloalkyl when n is 1 or 2; or, when n is 1, is COR₁₁, amino, mono-C₁-C₄alkylamino or di-C₁-C₄alkylamino;
R₇ is C₁-C₄alkyl; or C₁-C₄haloalkyl; unsubstituted phenyl or phenyl-C₁-C₂alkyl; or phenyl or phenyl-C₁-C₂alkyl which is substituted in the phenyl radical by halogen, C₁-C₄alkyl, C₁-C₄haloalkyl or C₁-C₄alkoxy;
R₈ is hydrogen, C₁-C₄alkyl; C₁-C₄haloalkyl; unsubstituted phenyl or phenyl-C₁-C₂alkyl; or phenyl or phenyl-C₁-C₂alkyl which is substituted in the phenyl radical by halogen, C₁-C₄alkyl, C₁-C₄haloalkyl or C₁-C₄alkoxy;
R₉ and R₁₀ are each independently of the other hydrogen or C₁-C₄alkyl; or
R₉ and R₁₀ together with the linking nitrogen atom are an unsubstituted or a C₁-C₄alkyl-substituted pyrrolidine or piperidine ring; and
R₁₁ is C₁-C₄alkyl; C₁-C₄haloalkyl; C₂-C₄alkoxy; phenyl which is substituted by halogen, C₁-C₄alkyl, C₁-C₄haloalkyl or C₁-C₄alkoxy; phenyl-C₁-C₂alkyl; or phenyl-C₁-C₂alkoxy.

16. A fluoran according to any one of claims 13 to 15, wherein
R₁ is hydrogen or methyl;
R₂ and R₃ are each independently of the other hydrogen; C₁-C₅alkyl; or
R₂ and R₃ together with the linking nitrogen atom are an unsubstituted pyrrolidine or piperidine ring;
R₄ is hydrogen or methyl;
R₅ is nitro; SO₂R₇; SO₂NR₉R₁₀; COR₁₁; CONR₉R₁₀; or C₁-C₄haloalkyl;
n is 0, 1, 2, 3 or 4;
R₆ is halogen when n is 1, 2, 3 or 4; or is nitro or methyl when n is 1 or 2; or, when n is 1, is nitro, amino, mono-C₁-C₄alkylamino or di-C₁-C₄alkylamino;
R₇ is C₁-C₄alkyl; or C₁-C₄haloalkyl; unsubstituted phenyl or phenyl which is substituted by halogen, C₁-C₄alkyl or C₁-C₄alkoxy;
R₉ and R₁₀ are each independently of the other hydrogen or C₁-C₄alkyl;
R₁₁ is C₁-C₄alkyl; C₂-C₄alkoxy; phenyl which is substituted by halogen, C₁-C₄alkyl, C₁-C₄haloalkyl or C₁-C₄alkoxy; phenyl-C₁-C₂alkyl or phenyl-C₁-C₂alkoxy.

17. A fluoran according to any one of claims 13 to 16, wherein R₅ is SO₂R₇.

18. A fluoran according to any one of claims 13 to 16, wherein R₅ is SO₂OR₈.

19. A fluoran according to any one of claims 13 to 16, wherein R₅ is SO₂NR₉R₁₀.

20. A fluoran according to any one of claims 13 to 16, wherein R₅ is COR₁₁.

21. A fluoran according to claim 20, wherein R₁₁ is C₁-C₄alkyl, C₂-C₄alkoxy or phenyl-C₁-C₂alkoxy.

22. A fluoran according to claim 20 or claim 21, wherein R₁₁ is C₂-C₄alkoxy or phenyl-C₁-C₂alkoxy.

23. A fluoran according to any one of claims 13 to 16, wherein R₅ is CONR₉R₁₀.

24. A fluoran according to any one of claims 13 to 16, wherein R₅ is C₁-C₄haloalkyl.

25. A fluoran according to any one of claims 13 to 16, wherein R₅ is nitro, and
R₂ and R₃ are each independently of the other hydrogen or C₄-C₈alkyl;
R₂ and R₃ together with the linking nitrogen atom are an unsubstituted or C₁-C₄alkyl-substituted pyrrolidine, piperidine, morpholine, thiomorpholine or piperazine ring.

26. A process for the preparation of a compound of the formula I according to any one of claims 13 to 25, which comprises reacting a benzophenone of the formula II with a phenol or phenol ether of the formula 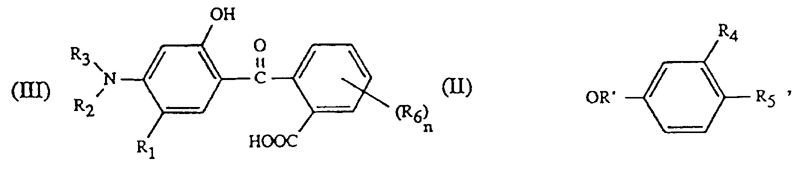 wherein R₁ to R₆ and n are as previously defined and R' is hydrogen or C₁-C₄alkyl, at temperatures of preferably from 0 to 70°C, in 50 to 100% sulfuric acid, to a phthalide of the formula 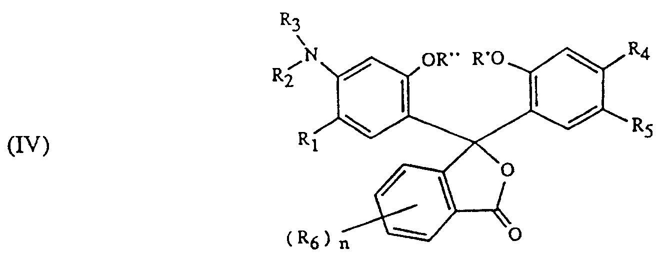 and subsequently effecting cyclisation at temperatures from 20 to 100°C to a compound of the formula I.

27. A process for the preparation of a sulfone of the formula I according to claim 17, which comprises reacting a sulfone ester of the formula I according to claim 18 with a carbanion or Grignard reagent of the general formula
R₇⁻M⁺ or R₇Mg-Hal, (V)
wherein
M⁺ is a cation equivalent of an alkali metal or alkaline earth metal ion; and
Hal is halogen, and R₇ is as previously defined.

28. A process for the preparation of a sulfonamide of the formula I according to claim 19, which comprises reacting a sulfone ester of the formula I according to claim 18 with an amine of the formula
HNR₉R₁₀, (VI)
wherein R₉ and R₁₀ are as previously defined.

29. A process for the preparation of a microencapsulated colour former, which comprises microencapsulating at least one compound of the formula I according to one or more of claims 1 to 12.

30. A process for the preparation of a microencapsulated colour former, which comprises microencapsulating at least one compound of the formula I according to one or more of claims 13 to 25.

31. A process for the preparation of a pressure-sensitive recording material, which comprises coating a first substrate with microcapsules comprising at least one compound of the formula I according to any one of claims 1 to 12, and applying a developer to the first substrate or to a second substrate which is in direct contact with the first substrate.

32. A process for the preparation of a pressure-sensitive recording material, which comprises coating a first substrate with microcapsules comprising a compound of the formula I according to any one of claims 13 to 25 and applying a developer to the first substrate or to a second substrate which is in direct contact with the first substrate.

33. A process for the preparation of a heat-sensitive recording material, which comprises coating a substrate with a mixture of colour formers comprising at least one compound of the formula I according to any one of claims 1 to 12 and with a developer.

34. A process for the preparation of a heat-sensitive recording material, which comprises coating a substrate with a mixture of colour formers comprising a compound of the formula I according to any one of claims 13 to 25 and with a developer.

35. A pressure-sensitive or heat-sensitive recording material comprising a mixture of colour formers with at least one compound of the formula I according to any one of claims 1 to 12 and a developer.

36. A pressure-sensitive or heat-sensitive recording material comprising a mixture of colour formers with at least one compound of the formula I according to any one of claims 13 to 25 and a developer.

## Revendications

1. Support d'enregistrement sensible à la pression ou à la chaleur, contenant au moins un composé de formule 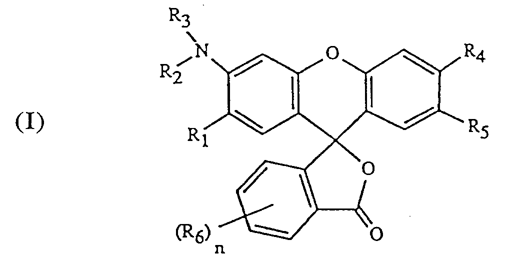 dans laquelle
R₁ représente un atome d'hydrogène ou un groupe alkyle en C₁₋₄,
R₂ et R₃ représentent, indépendamment l'un de l'autre, un atome d'hydrogène, un groupe alkyle en C₁₋₈, un groupe cycloalkyle en C₄₋₇ non substitué ou portant un substituant alkyle en C₁₋₄ ou halogéno, un groupe phényle non substitué ou portant des substituants alkyle en C₁₋₄, hydroxy ou halogéno, un groupe phényl-(alkyle en C₁₋₄), alcényle en C₃₋₆, alcoxy en C₁₋₄, (alcoxy en C₁₋₄)-(alkyle en C₁₋₄), 2-tétrahydrofuranyle ou
R₂ et R₃ forment avec l'atome d'azote auquel ils sont liés un cycle pyrrolidino, pipéridino, morpholino, thiomorpholino ou pipérazino non substitué ou portant un groupe alkyle en C₁₋₄,
R₄ représente un atome d'hydrogène, un résidu hydroxy ou alkyle en C₁₋₄,
R₅ représente un groupe nitro, SO₂-R₇, SO₂OR₈, SO₂NR₉R₁₀, COR₁₁, CONR₉R₁₀, halogénoalkyle en C₁₋₄, ou un résidu 2-triazinyle ou 1-benzotriazolyle non substitué ou portant un substituant halogéno ou hydroxy,
R₆ représente un atome d'hydrogène, un groupe nitro, alkyle en C₁₋₄, halogénoalkyle en C₁₋₄, amino, mono-(alkyle en C₁₋₄)-amino, di-(alkyle en C₁₋₄)-amino, ou COR₁₁,
n vaut 0, 1, 2, 3 ou 4,
R₇ représente un résidu alkyle en C₁₋₈ ou halogénoalkyle en C₁₋₈, un résidu phényle ou phényl-(alkyle en C₁₋₄) non substitué ou portant, sur le noyau phényle, des substituants halogéno, alkyle en C₁₋₄, halogénoalkyle en C₁₋₄ ou alcoxy en C₁₋₄,
R₈ représente un atome d'hydrogène, un groupe alkyle en C₁₋₈, un groupe halogénoalkyle en C₁₋₈, un résidu phényle ou phényl-(alkyle en C₁₋₄) non substitué ou portant, sur le noyau phényle, des substituants halogéno, alkyle en C₁₋₄, halogénoalkyle en C₁₋₄ ou alcoxy en C₁₋₄,
R₉ et R₁₀ représentent, indépendamment l'un de l'autre, un atome d'hydrogène ou un groupe alkyle en C₁₋₈, ou R₉ et R₁₀ forment avec l'atome d'azote auquel ils sont liés un cycle pyrrolidino, pipéridino, morpholino, thiomorpholino ou pipérazino non substitué ou portant un groupe alkyle en C₁₋₄,
R₁₁ représente un groupe hydroxyle, alkyle en C₁₋₈, alcoxy en C₂₋₈, halogénoalkyle en C₁₋₈, phényle portant des substituants halogéno, alkyle en C₁₋₄, halogénoalkyle en C₁₋₄, alcoxy en C₁₋₄, un groupe phényl-(alkyle en C₁₋₄) ou phényl-(alcoxy en C₁₋₄),
à l'exclusion du 3-diéthylamino-7-méthylsulfonylfluorane, 3-diéthylamino-7-nitrofluorane, 3-diéthylamino-6-méthyl-7-nitrofluorane, acide 3-diéthylamino-fluorane-7-sulfonique, 3-diéthylamino-7-(4-éthoxyphényl)sulfonylfluorane et 3-diéthylamino-7-benzyloxyfluorane.

2. Support d'enregistrement conforme à la revendication 1 contenant au moins un composé chromogène de formule (I) dans laquelle
R₁ représente un atome d'hydrogène ou un groupe alkyle en C₁₋₄,
R₂ et R₃ représentent, indépendamment l'un de l'autre, un atome d'hydrogène ou un groupe alkyle en C₁₋₅, ou R₂ et R₃ forment avec l'atome d'azote auquel ils sont liés un cycle pyrrolidino ou pipéridino non substitué ou portant un groupe alkyle en C₁₋₄,
R₄ représente un atome d'hydrogène ou un résidu alkyle en C₁₋₄,
R₅ représente un groupe nitro, SO₂-R₇, SO₂OR₈, SO₂NR₉R₁₀, COR₁₁, CONR₉R₁₀ ou halogénoalkyle en C₁₋₄,
n vaut 0, 1, 2, 3 ou 4,
R₆, lorsque n vaut 1, 2, 3 ou 4, représente un atome d'halogène, lorsque n vaut 1 ou 2, représente un groupe alkyle en C₁₋₄ ou halogénoalkyle en C₁₋₄, ou, lorsque n vaut 1, représente un groupe nitro, COR₁₁, amino, mono-(alkyle en C₁₋₄)-amino ou di-(alkyle en C₁₋₄)-amino,
R₇ représente un résidu alkyle en C₁₋₄ ou halogénoalkyle en C₁₋₄, un résidu phényle ou phényl-(alkyle en C₁₋₂) non substitué ou portant, sur le noyau phényle, des substituants halogéno, alkyle en C₁₋₄, halogénoalkyle en C₁₋₄ ou alcoxy en C₁₋₄,
R₈ représente un atome d'hydrogène, un groupe alkyle en C₁₋₄, un groupe halogénoalkyle en C₁₋₄, un résidu phényle ou phényl-(alkyle en C₁₋₄) non substitué ou portant, sur le noyau phényle, des substituants halogéno, alkyle en C₁₋₄, halogénoalkyle en C₁₋₄ ou alcoxy en C₁₋₄,
R₉ et R₁₀ représentent, indépendamment l'un de l'autre, un atome d'hydrogène ou un groupe alkyle en C₁₋₄, ou R₉ et R₁₀ forment avec l'atome d'azote auquel ils sont liés un cycle pyrrolidino ou pipéridino non substitué ou portant un groupe alkyle en C₁₋₄,
R₁₁ représente un groupe alkyle en C₁₋₄, halogénoalkyle en C₁₋₄, alcoxy en C₂₋₄, phényle portant des substituants halogéno, alkyle en C₁₋₄, halogénoalkyle en C₁₋₄, alcoxy en C₁₋₄, un groupe phényl-(alkyle en C₁₋₂) ou phényl-(alcoxy en C₁₋₂).

3. Support d'enregistrement conforme à la revendication 1 ou 2, contenant au moins un composé chromogène de formule (I) dans laquelle
R₁ représente un atome d'hydrogène ou un groupe méthyle,
R₂ et R₃ représentent, indépendamment l'un de l'autre, un atome d'hydrogène ou un groupe alkyle en C₁₋₅, ou R₂ et R₃ forment avec l'atome d'azote auquel ils sont liés un cycle pyrrolidino ou pipéridino non substitué,
R₄ représente un atome d'hydrogène ou un résidu méthyle,
R₅ représente un groupe nitro, SO₂-R₇, SO₂NR₉R₁₀, COR₁₁, CONR₉R₁₀ ou halogénoalkyle en C₁₋₄,
n vaut 0, 1, 2, 3 ou 4,
R₆, lorsque n vaut 1, 2, 3 ou 4, représente un atome d'halogène, et lorsque n vaut 1 ou 2, représente un groupe méthyle; ou, lorsque n vaut 1 représente un groupe nitro, amino, mono-(alkyle en C₁₋₄)-amino, di-(alkyle en C₁₋₄)-amino,
R₇ représente un résidu alkyle en C₁₋₄ ou halogénoalkyle en C₁₋₄, un résidu phényle non substitué ou portant, sur le noyau phényle, des substituants halogéno, alkyle en C₁₋₄ ou alcoxy en C₁₋₄,
R₉ et R₁₀ représentent, indépendamment l'un de l'autre, un atome d'hydrogène ou un groupe alkyle en C₁₋₄,
R₁₁ représente un groupe alkyle en C₁₋₄, alcoxy en C₂₋₄, phényle portant des substituants halogéno, alkyle en C₁₋₄, halogénoalkyle en C₁₋₄ ou alcoxy en C₁₋₄, un groupe phényl-(alkyle en C₁₋₂) ou phényl-(alcoxy en C₁₋₂).

4. Support d'enregistrement conforme à une des revendications 1 à 3 contenant au moins un composé chromogène de formule (I) dans lequel R₅ représente un groupe SO₂R₇.

5. Support d'enregistrement conforme à une des revendications 1 à 3 contenant au moins un composé chromogène de formule (I) dans lequel R₅ représente un groupe SO₂OR₈.

6. Support d'enregistrement conforme à une des revendications 1 à 3 contenant au moins un composé chromogène de formule (I) dans lequel R₅ représente un groupe SO₂NR₉R₁₀.

7. Support d'enregistrement conforme à une des revendications 1 à 3 contenant au moins un composé chromogène de formule (I) dans lequel R₅ représente un groupe COR₁₁.

8. Support d'enregistrement conforme à la revendication 7 caractérisé en ce que R₁₁ représente un groupe alkyle en C₁₋₄, alcoxy en C₂₋₄ ou phényl-(alcoxy en C₁₋₂).

9. Support d'enregistrement conforme à la revendication 7 ou 8 caractérisé en ce que R₁₁ représente un groupe alcoxy en C₂₋₄ ou phényl-(alcoxy en C₁₋₂).

10. Support d'enregistrement conforme à une des revendications 1 à 3 contenant au moins un composé chromogène de formule (I) dans lequel R₅ représente un groupe CONR₉R₁₀.

11. Support d'enregistrement conforme à une des revendications 1 à 3 contenant au moins un composé chromogène de formule (I) dans lequel R₅ représente un groupe halogénoalkyle en C₁₋₄.

12. Support d'enregistrement conforme à une des revendications 1 à 3 contenant au moins un composé chromogène de formule (I) dans lequel R₅ représente un groupe nitro.

13. Fluoranes de formule générale 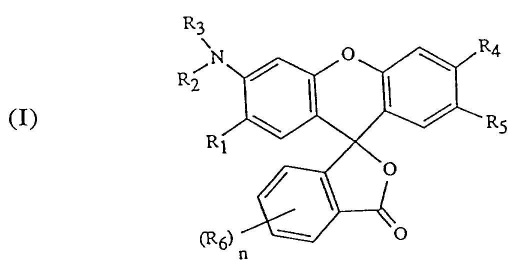 dans laquelle
R₁ représente un atome d'hydrogène ou un groupe alkyle en C₁₋₄,
R₂ et R₃ représentent, indépendamment l'un de l'autre, un atome d'hydrogène, un groupe alkyle en C₁₋₈, un groupe cycloalkyle en C₄₋₇ non substitué ou portant un substituant alkyle en C₁₋₄ ou halogéno, un groupe phényle non substitué ou portant des substituants alkyle en C₁₋₄, hydroxy ou halogéno, un groupe phényl-(alkyle en C₁₋₄), alcényle en C₃₋₆, alcoxy en C₁₋₄, (alcoxy en C₁₋₄)-(alkyle en C₁₋₄), 2-tétrahydrofuranyle ou R₂ et R₃ forment avec l'atome d'azote auquel ils sont liés un cycle pyrrolidino, pipéridino, morpholino, thiomorpholino ou pipérazino non substitué ou portant un groupe alkyle en C₁₋₄,
R₄ représente un atome d'hydrogène, un résidu hydroxy ou alkyle en C₁₋₄,
R₅ représente un groupe nitro, SO₂-R₇, SO₂OR₈, SO₂NR₉R₁₀, COR₁₁, CONR₉R₁₀, halogénoalkyle en C₁₋₄, ou un résidu 2-triazinyle ou 1-benzotriazolyle non substitué ou portant un substituant halogéno ou hydroxy,
R₆ représente un atome d'halogène, un groupe nitro, alkyle en C₁₋₄, halogénoalkyle en C₁₋₄, amino, mono-(alkyle en C₁₋₄)-amino, di-(alkyle en C₁₋₄)-amino, ou COR₁₁,
n vaut 0, 1, 2, 3 ou 4,
R₇ représente un résidu alkyle en C₁₋₈ ou halogénoalkyle en C₁₋₈, un résidu phényle ou phényl-(alkyle en C₁₋₄) non substitué ou portant, sur le noyau phényle, des substituants halogéno, alkyle en C₁₋₄, halogénoalkyle en C₁₋₄ ou alcoxy en C₁₋₄,
R₈ représente un atome d'hydrogène, un groupe alkyle en C₁₋₈, un groupe halogénoalkyle en C₁₋₈, un résidu phényle ou phényl-(alkyle en C₁₋₄) non substitué ou portant, sur le noyau phényle, des substituants halogéno, alkyle en C₁₋₄, halogénoalkyle en C₁₋₄ ou alcoxy en C₁₋₄,
R₉ et R₁₀ représentent, indépendamment l'un de l'autre, un atome d'hydrogène ou un groupe alkyle en C₁₋₈, ou R₉ et R₁₀ forment avec l'atome d'azote auquel ils sont liés un cycle pyrrolidino, pipéridino, morpholino, thiomorpholino ou pipérazino non substitué ou portant un groupe alkyle en C₁₋₄,
R₁₁ représente un groupe hydroxyle, alkyle en C₁₋₈, alcoxy en C₂₋₈, halogénoalkyle en C₁₋₈, phényle portant des substituants halogéno, alkyle en C₁₋₄, halogénoalkyle en C₁₋₄, alcoxy en C₁₋₄, un groupe phényl-(alkyle en C₁₋₄) ou phényl-(alcoxy en C₁₋₄),
à l'exclusion du 3-diéthylamino-7-méthylsulfonylfluorane, du 3-diéthylamino-7-nitrofluorane, du 3-diéthylamino-6-méthyl-7-nitrofluorane et de l'acide 3-diéthylamino-fluorane-7-sulfonique.

14. Fluoranes conformes à la revendication 13 dans lesquels
R₁ représente un atome d'hydrogène ou un groupe alkyle en C₁₋₄,
R₂ et R₃ représentent, indépendamment l'un de l'autre, un atome d'hydrogène ou un groupe alkyle en C₁₋₅, ou R₂ et R₃ forment avec l'atome d'azote auquel ils sont liés un cycle pyrrolidino ou pipéridino non substitué ou portant un groupe alkyle en C₁₋₄,
R₄ représente un atome d'hydrogène ou un résidu alkyle en C₁₋₄,
R₅ représente un groupe nitro, SO₂R₇, SO₂OR₈, SO₂NR₉R₁₀, COR₁₁, CONR₉R₁₀ ou halogénoalkyle en C₁₋₄,
n vaut 0, 1, 2, 3 ou 4,
R₆, lorsque n vaut 1, 2, 3 ou 4, représente un atome d'halogène, lorsque n vaut 1 ou 2, représente un groupe alkyle en C₁₋₄ ou halogénoalkyle en C₁₋₄, ou, lorsque n vaut 1 représente un groupe nitro, COR₁₁, amino, mono-(alkyle en C₁₋₄)-amino ou di-(alkyle en C₁₋₄)-amino,
R₇ représente un résidu alkyle en C₁₋₄ ou halogénoalkyle en C₁₋₄, un résidu phényle ou phényl-(alkyle en C₁₋₂) non substitué ou portant, sur le noyau phényle, des substituants halogéno, alkyle en C₁₋₄, halogénoalkyle en C₁₋₄ ou alcoxy en C₁₋₄,
R₈ représente un atome d'hydrogène, un groupe alkyle en C₁₋₄, un groupe halogénoalkyle en C₁₋₄, un résidu phényle ou phényl-(alkyle en C₁₋₂) non substitué ou portant, sur le noyau phényle, des substituants halogéno, alkyle en C₁₋₄, halogénoalkyle en C₁₋₄ ou alcoxy en C₁₋₄,
R₉ et R₁₀ représentent, indépendamment l'un de l'autre, un atome d'hydrogène ou un groupe alkyle en C₁₋₄, ou R₉ et R₁₀ forment avec l'atome d'azote auquel ils sont liés un cycle pyrrolidino ou pipéridino non substitué ou portant un groupe alkyle en C₁₋₄,
R₁₁ représente un groupe alkyle en C₁₋₄, alcoxy en C₂₋₄, halogénoalkyle en C₁₋₄, phényle portant des substituants halogéno, alkyle en C₁₋₄, halogénoalkyle en C₁₋₄, alcoxy en C₁₋₄, un groupe phényl-(alkyle en C₁₋₂) ou phényl-(alcoxy en C₁₋₂).

15. Fluoranes conformes à la revendication 13 ou 14 dans lesquels
R₁ représente un atome d'hydrogène ou un groupe alkyle en C₁₋₄,
R₂ et R₃ représentent, indépendamment l'un de l'autre, un atome d'hydrogène ou un groupe alkyle en C₁₋₅, ou
R₂ et R₃ forment avec l'atome d'azote auquel ils sont liés un cycle pyrrolidino ou pipéridino non substitué ou portant un groupe alkyle en C₁₋₄,
R₄ représente un atome d'hydrogène ou un résidu alkyle en C₁₋₄,
R₅ représente un groupe nitro, SO₂R₇, SO₂OR₈, SO₂NR₉R₁₀, COR₁₁, CONR₉R₁₀ ou halogénoalkyle en C₁₋₄,
n vaut 0, 1, 2, 3 ou 4,
R₆, lorsque n vaut 1, 2, 3 ou 4, représente un atome d'halogène, et lorsque n vaut 1 ou 2, représente un groupe nitro, alkyle en C₁₋₄ ou halogénoalkyle en C₁₋₄; ou, lorsque n vaut 1, représente un groupe COR₁₁, nitro, amino, mono-(alkyle en C₁₋₄)-amino ou di-(alkyle en C₁₋₄)-amino,
R₇ représente un résidu alkyle en C₁₋₄ ou halogénoalkyle en C₁₋₄, un résidu phényle ou phényl-(alkyle en C₁₋₂) non substitué ou portant, sur le noyau phényle, des substituants halogéno, alkyle en C₁₋₄ ou alcoxy en C₁₋₄,
R₈ représente un atome d'hydrogène, un groupe alkyle en C₁₋₄, un groupe halogénoalkyle en C₁₋₄, un résidu phényle ou phényl-(alkyle en C₁₋₂) non substitué ou portant, sur le noyau phényle, des substituants halogéno, alkyle en C₁₋₄, halogénoalkyle en C₁₋₄ ou alcoxy en C₁₋₄,
R₉ et R₁₀ représentent indépendamment l'un de l'autre un atome d'hydrogène ou un groupe alkyle en C₁₋₄, ou R₉ et R₁₀ forment avec l'atome d'azote auquel ils sont liés un cycle pyrrolidino ou pipéridino non substitué ou portant un groupe alkyle en C₁₋₄,
R₁₁ représente un groupe alkyle en C₁₋₄, halogénoalkyle en C₁₋₄, alcoxy en C₂₋₄, phényle portant des substituants halogéno, alkyle en C₁₋₄, halogénoalkyle en C₁₋₄ ou alcoxy en C₁₋₄, un groupe phényl-(alkyle en C₁₋₂) ou phényl-(alcoxy en C₁₋₂).

16. Fluoranes conformes à une des revendicaitons 13 à 15 dans lesquels
R₁ représente un atome d'hydrogène ou un groupe méthyle,
R₂ et R₃ représentent, indépendamment l'un de l'autre, un atome d'hydrogène, un groupe alkyle en C₁₋₅, ou
R₂ et R₃ forment avec l'atome d'azote auquel ils sont liés un cycle pyrrolidino ou pipéridino non substitué,
R₄ représente un atome d'hydrogène ou un résidu méthyle,
R₅ représente un groupe nitro, SO₂R₇, SO₂NR₉R₁₀, COR₁₁, CONR₉R₁₀ ou halogénoalkyle en C₁₋₄,
n vaut 0, 1, 2, 3 ou 4,
R₆, lorsque n vaut 1, 2, 3 ou 4, représente un atome d'halogène, et lorsque n vaut 1 ou 2, représente un groupe nitro ou méthyle; ou, lorsque n vaut 1, représente un groupe nitro, amino, mono-(alkyle en C₁₋₄)-amino ou di-(alkyle en C₁₋₄)-amino,
R₇ représente un résidu alkyle en C₁₋₄ ou halogénoalkyle en C₁₋₄, un résidu phényle non substitué ou portant, sur le noyau phényle, des substituants halogéno, alkyle en C₁₋₄ ou alcoxy en C₁₋₄,
R₉ et R₁₀ représentent, indépendamment l'un de l'autre, un atome d'hydrogène ou un groupe alkyle en C₁₋₄,
R₁₁ représente un groupe alkyle en C₁₋₄, alcoxy en C₂₋₄, phényle portant des substituants halogéno, alkyle en C₁₋₄, halogénoalkyle en C₁₋₄ ou alcoxy en C₁₋₄, un groupe phényl-(alkyle en C₁₋₂) ou phényl-(alcoxy en C₁₋₂).

17. Fluoranes conformes à une des revendications 13 à 16 dans lesquels R₅ représente un groupe SO₂R₇.

18. Fluoranes conformes à une des revendications 13 à 16 dans lesquels R₅ représente un groupe SO₂OR₈.

19. Fluoranes conformes à une des revendications 13 à 16 dans lesquels R₅ représente un groupe SO₂NR₉R₁₀.

20. Fluoranes conformes à une des revendications 13 à 16 dans lesquels R₅ représente un groupe COR₁₁.

21. Fluoranes conformes à la revendication 20, caractérisés en ce que R₁₁ représente un groupe alkyle en C₁₋₄, alcoxy en C₂₋₄ ou phényl-(alcoxy en C₁₋₂).

22. Fluoranes conformes à la revendication 20 ou 21, caractérisés ence que R₁₁ représente un groupe alcoxy en C₂₋₄ ou phényl-(alcoxy en C₁₋₂).

23. Fluoranes conformes à une des revendications 13 à 16 dans lesquels R₅ représente un groupe CONR₉R₁₀.

24. Fluoranes conformes à une des revendications 13 à 16 dans lesquels R₅ représente un groupe halogénoalkyle en C₁₋₄.

25. Fluoranes conformes à une des revendications 13 à 16 dans lesquels
R₅ représente un groupe nitro; et
R₂ et R₃ représentent, indépendamment l'un de l'autre, un atome d'hydrogène ou un groupe alkyle en C₄₋₈, ou R₂ et R₃ forment avec l'atome d'azote auquel ils sont liés un cycle pyrrolidino, pipéridino, morpholino, thiomorpholino ou pipérazino non substitué ou substitué par un groupe alkyle en C₁₋₄.

26. Procédé de préparation de composés de formule (I) conformes à une des revendications 13 à 25, caractérisé en ce que l'on fait réagir une benzophénone de formule (II) avec un phénol ou un éther de phénol de formule (III) 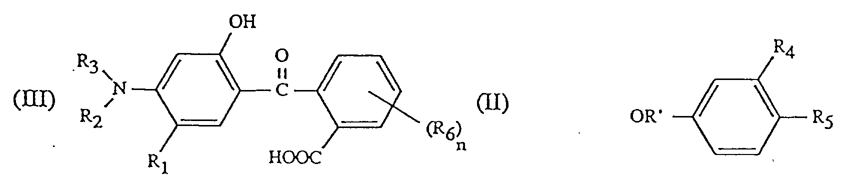 dans lesquelles les résidus R₁ à R₆ et n ont la signification indiquée précédemment, et R' représente un atome d'hydrogène ou un groupe alkyle en C₁₋₄, à des températures comprises de préfrence entre 0 et 70 °C, dans de l'acide sulfurique à 50 - 100 %, pour obtenir un phtalide de formule 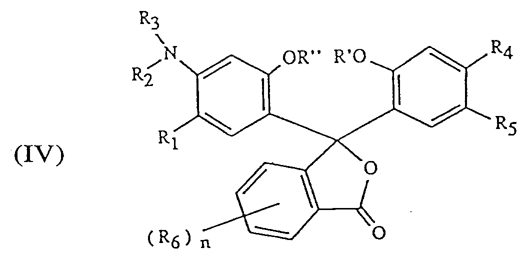 cette réaction étant suivie d'une cyclisation à des températures comprises entre 20 et 100 °C ce qui aboutit au composé de formule I.

27. Procédé de préparation d'une sulfone de formule (I) conforme à la revendication 17, par réaction d'un ester sulfonate de formule (I) conforme à la revendication 18 avec un carbanion ou un réactif de Grignard de formule générale
R₇⁻M⁺ ou R₇Mg-Hal (V)
dans laquelle M⁺ représente un équivalent d'un cation d'un métal alcalin ou alcalino-terreux et Hal représente un atome d'halogène, et R₇ est défini comme ci-dessus.

28. Procédé de préparation d'un sulfonarnide de formule (I) conforme à la revendication 19 par réaction d'un ester sulfonate de formule I conforme à la revendication 18, avec une amine de formule
HNR₉R₁₀ (VI)
où R₉ et R₁₀ sont définis comme ci-dessus.

29. Procédé de préparation de composés chromogènes enfermés dans des microcapsules, caractérisé en ce que l'on enferme au moins un composé de formule (I) conforme à une ou plusieurs des revendications 1 à 12 dans des microcapsules.

30. Procédé de préparation de composés chromogènes enfermés dans des microcapsules, caractérisé en ce que l'on enferme au moins un composé de formule I conforme à une ou plusieurs des revendications 13 à 25 dans des microcapsules.

31. Procédé de préparation d'un Support d'enregistrement sensible à la pression, caractérisé en ce que l'on applique, sur un premier support, des microcapsules contenant au moins un composé de formule I conforme à une des revendications 1 à 12 et sur le premier support ou sur un deuxième support, directement en contact avec le premier, un agent révélateur.

32. Procédé de préparation d'un support d'enregistrement sensible à la pression, caractérisé en ce que l'on applique sur un premier support des microcapsules contenant un composé de formule I conforme à une des revendications 13 à 25 et sur le premier support ou sur un deuxième support, directement en contact avec le premier, un agent révélateur.

33. Procédé de préparation d'un support d'enregistrement thermosensible, caractérisé en ce que l'on applique sur un support un mélange de composés chromogènes contenant au moins un composé de formule I conforme à une des revendications 1 à 12, et un agent révélateur.

34. Procédé de préparation d'un support d'enregistrement thermosensible, caractérisé en ce que l'on applique sur un support un mélange de composés chromogènes contenant un composé de formule I conforme à une des revendications 13 à 25, et un agent révélateur.

35. Support d'enregistrement sensible à la pression ou à la chaleur contenant un mélange de composés chromogènes renfermant au moins un composé de formule I conforme à une des revendications 1 à 12 et un agent révélateur.

36. Support d'enregistrement sensible à la pression ou à la chaleur contenant un mélange de composés chromogènes renfermant au moins un composé de formule I conforme à une des revendications 13 à 25 et un agent révélateur.
